# EUROPEAN PATENT APPLICATION

(11) **EP 4 516 771 A1**
(43) Date of publication of application: **05.03.2025**
(21) Application number: 23795619.8
(22) Date of filing: 28.04.2023
(51) Int. Cl.: C07C 49/577, C07C 49/533, C07C 49/537, C07C 49/543, C07C 49/547, C07C 49/557, A61K 31/12, A61K 31/075, A61P 35/00, A61P 13/08

(54) **COMPOUND HAVING ANTI-ANDROGEN RECEPTOR ACTIVITY, AND USE THEREOF**

(30) Priority: 29.04.2022 CN 202210476508
(71) Applicant: Nanjing Minova Pharmaceutical Co., Ltd., Nanjing, Jiangsu 210046 (CN)
(72) Inventor: LIU, Fei, Nanjing, Jiangsu 210046 (CN); WU, Gang, Nanjing, Jiangsu 210046 (CN); WANG, Xiaobo, Nanjing, Jiangsu 210046 (CN); SONG, Wenqi, Nanjing, Jiangsu 210046 (CN); ZHANG, Wanchao, Nanjing, Jiangsu 210046 (CN); CHEN, Qing, Nanjing, Jiangsu 210046 (CN); SUN, Yuting, Nanjing, Jiangsu 210046 (CN); ZHA, Quanwen, Nanjing, Jiangsu 210046 (CN); KANG, Le, Nanjing, Jiangsu 210046 (CN)
(74) Representative: Gille Hrabal Partnerschaftsgesellschaft mbB Patentanwälte
(86) International application number: PCT/CN2023/091555
(87) International publication number: WO 2023/208187

(57) **Abstract**

Provided in the present disclosure are a compound represented by formula **I,** and a racemate, stereoisomer, tautomer, solvate, polymorph, or pharmaceutically acceptable salts thereof. The compound has good prostate tumor cell proliferation inhibitory activity and anti-AR activity. The compound has good in-vivo metabolic properties, has high AUC and Cₘₐₓ, is good in druggability, and can be used for preventing and/or treating androgen-related disorders. The compound provided by the invention also has a good hair growth promoting effect, and can effectively increase the number of hair follicles and the growth length of hair.

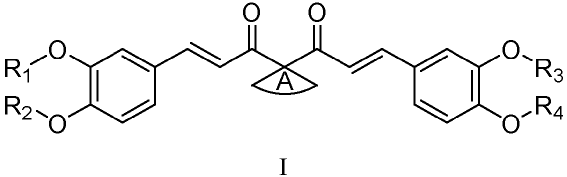

## Description

The present application claims priority to a prior application with the patent application No. 202210476508.3, entitled "COMPOUND HAVING ANTI-ANDROGEN RECEPTOR ACTIVITY, AND USE THEREOF" and filed with the China National Intellectual Property Administration on April 29, 2022, which is incorporated herein by reference in its entirety.

### TECHNICAL FIELD

The present disclosure belongs to the technical field of medicines, and particularly relates to a compound having anti-androgen receptor activity and use thereof.

### BACKGROUND

The androgen receptor (AR) is a member of a large family of ligand-dependent transcription factors, which are referred to as the steroid receptor superfamily. The androgen receptor is a steroid hormone receptor having a ligand binding region, a DNA binding region, and a plurality of phosphorylation sites. The AR binds to a ligand (androgen) *in vivo* to form an AR dimer, which is then phosphorylated and transferred from the cytoplasm to the cell nucleus, thereby mediating the transcription and activation of various pathways in the cell nucleus.

The AR is widely distributed in numerous human body parts and organs, and plays a vital role in the progression of numerous androgen-related diseases such as cancers, alopecia, acne, and the like.

Androgens and the AR have been reported in the document to play an important role in the growth of normal prostate and prostate cancer, and anti-androgen drugs have been widely used in the treatment of prostate cancer. In addition, high serum androgen levels are associated with acne and androgenetic alopecia, and anti-androgen therapy has a potential impact on acne and androgenetic alopecia. However, androgens are involved in a number of biological processes. Blocking or inhibiting the binding of androgens to their respective receptors results in increased levels of effective androgens in the surrounding environment, and the increased levels of androgens in the surrounding environment can affect other androgen-related biological processes and can cause undesirable side effects. Therefore, currently, inhibiting the production of androgen receptors has become a research focus, and compounds having anti-androgen receptor activity have been used as potential therapies for androgen receptor-related conditions.

Chinese Patent Application CN03808650.6 discloses a class of curcumin analogs having anti-androgen receptor production activity, which can effectively inhibit the production of androgen receptors in cells. However, most of these curcumin analogs are oily substances or have undesirable activity.

Mental diseases such as self-esteem reduction or depression, etc. often plague patients with alopecia, skin diseases, or other diseases, causing serious psychological burdens to the patients. Therefore, a new class of compounds having good druggability, bioactivity, and other properties are urgently needed.

### SUMMARY

The present disclosure provides a curcumin derivative that has high bioavailability and good bioactivity, acts fast, and can maintain a stable physiological concentration *in vivo* for a long time.

The present disclosure is also intended to provide a composition comprising the curcumin derivative described above and use of the curcumin derivative described above for manufacturing a medicament for the treatment, prevention, or amelioration of symptoms or diseases from androgen-related disorders.

The objectives of the present disclosure are achieved by the following technical solutions.

In a first aspect, the present disclosure provides a compound represented by the following formula I, a racemate thereof, a stereoisomer thereof, a tautomer thereof, a solvate thereof, a polymorph thereof or a pharmaceutically acceptable salt thereof, 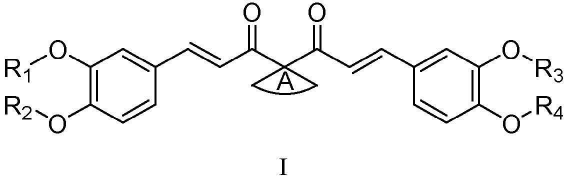
wherein R₁, R₂, R₃, and R₄ are identical or different and are each independently selected from the following groups that are unsubstituted or optionally substituted with one, two or more Ra: C₁₋₁₂ alkyl or deuterated C₁₋₁₂ alkyl; 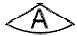
represents the following ring systems that are unsubstituted or optionally substituted with one, two or more Rb: C₃₋₂₀ cycloalkyl, C₄₋₂₀ cycloalkenyl, 3- to 20-membered heterocyclyl, a spiro ring formed from C₃₋₂₀ cycloalkyl and C₃₋₂₀ cycloalkyl, a spiro ring formed from C₃₋₂₀ cycloalkyl and 3- to 20-membered heterocyclyl, and a spiro ring formed from 3- to 20-membered heterocyclyl and 3- to 20-membered heterocyclyl;
Ra are identical or different and are each independently selected from halogen, =O, hydroxyl, amino, C₁₋₁₂ alkyl, and C₁₋₁₂ alkoxy;
Rb are identical or different and are each independently selected from deuterium, halogen, =O, hydroxyl, amino, and the following groups that are unsubstituted or optionally substituted with one, two or more Rc: C₁₋₁₂ alkyl, C₁₋₁₂ alkoxy, C₃₋₂₀ cycloalkyl, 3- to 20-membered heterocyclyl, -NHC₁₋₁₂ alkyl, -N(C₁₋₁₂ alkyl)₂, -NHCOC₁₋₁₂ alkyl, -CONHC₁₋₁₂ alkyl, -NHC₃₋₂₀ cycloalkyl, -NHCOC₃₋₂₀ cycloalkyl, -CONHC₃₋₂₀ cycloalkyl, -S(O)₂C₁₋₁₂ alkyl, and -COOC₁₋₁₂ alkyl; or two Rb attached to the same carbon atom, together with the carbon atom attached thereto, form the following ring systems that are unsubstituted or optionally substituted with one, two or more Rc: C₃₋₂₀ cycloalkyl and 3- to 20-membered heterocyclyl;
Rc is selected from halogen, hydroxyl, amino, C₁₋₁₂ alkyl, C₁₋₁₂ alkoxy, C₃₋₂₀ cycloalkyl, 3- to 20-membered heterocyclyl, and 5- to 20-membered heteroaryl.

According to an embodiment of the present disclosure, R₁, R₂, R₃, and R₄ are identical or different and are each independently selected from C₁₋₆ alkyl and deuterated C₁₋₆ alkyl.

According to an embodiment of the present disclosure, 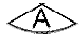 represents the following ring systems that are unsubstituted or optionally substituted with one, two or more (e.g., 1, 2, 3, 4, or 5) Rb: C₃₋₁₂ cycloalkyl, C₄₋₁₂ cycloalkenyl, 3- to 12-membered heterocyclyl, a spiro ring formed from C₃₋₁₂ cycloalkyl and C₃₋₁₂ cycloalkyl, a spiro ring formed from C₃₋₁₂ cycloalkyl and 3- to 12-membered heterocyclyl, and a spiro ring formed from 3- to 12-membered heterocyclyl and 3- to 12-membered heterocyclyl.

According to an embodiment of the present disclosure, Rb are identical or different and are each independently selected from deuterium, halogen, =O, hydroxyl, amino, and the following groups that are unsubstituted or optionally substituted with one, two or more (e.g., 1, 2, 3, 4, or 5) Rc: C₃₋₁₂ cycloalkyl, 3- to 12-membered heterocyclyl, -NHC₁₋₆ alkyl, -N(C₁₋₆ alkyl)₂, -NHCOC₁₋₆ alkyl, - NHC₃₋₁₂ cycloalkyl, -S(O)₂C₁₋₆ alkyl, and -COOC₁₋₆ alkyl.

In some embodiments of the present disclosure, Rc is selected from halogen, hydroxyl, C₃₋₁₂ cycloalkyl, 3- to 12-membered heterocyclyl, and 5- to 12-membered heteroaryl.

In some embodiments of the present disclosure, the 3- to 20-membered heterocyclyl may be nitrogen-containing heterocyclyl, oxygen-containing heterocyclyl, etc., for example, piperidyl, piperazinyl, morpholinyl, dioxanyl, etc.

In some embodiments of the present disclosure, 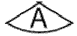 may be the following ring systems that are unsubstituted or optionally substituted with one, two or more Rb: C₃₋₁₀ monocyclic cycloalkyl, C₃₋₁₀ monocyclic cycloalkenyl, oxa C₂₋₁₀ monocyclic cycloalkyl, aza C₂₋₁₀ monocyclic cycloalkyl, spiro C₆₋₁₆ alkyl, oxaspiro C₆₋₁₆ alkyl; for example, spirohexyl, spiroheptyl, spirooctyl, spirononyl, spirodecyl, azacyclopentane, azacyclohexane, azacycloheptane, oxaspirohexyl, oxaspiroheptyl, oxaspirooctyl, oxaspirononyl, or oxaspirodecyl.

In some embodiments of the present disclosure, 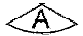 may be the following ring systems that are unsubstituted or optionally substituted with one, two or more Rb: cyclopropyl, cyclobutyl, cyclopentyl, cyclohexyl, cyclohexenyl, spiro[2.3]hexanyl, 1,3-dioxanyl, azacyclohexane, and 1,4-dioxaspiro[4,5]decanyl.

In some embodiments of the present disclosure, Rb is selected from deuterium, F, Cl, Br, I, =O, hydroxyl, amino, tert-butoxycarbonyl, -S(O)₂CH₃, -COOC(CH₃)₃, 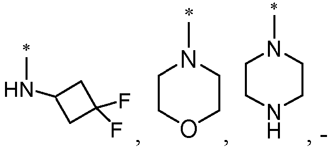 N(C₂H₅)₂, -N(CH₃)₂,

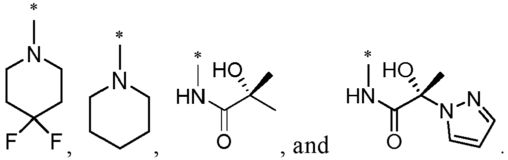

In some embodiments of the present disclosure, R₁, R₂, R₃, and R₄ are identical or different and are each independently selected from C₁₋₃ alkyl and deuterated C₁₋₃ alkyl, such as methyl or deuterated methyl.

In some embodiments of the present disclosure, the compound represented by formula I has a structure shown below: 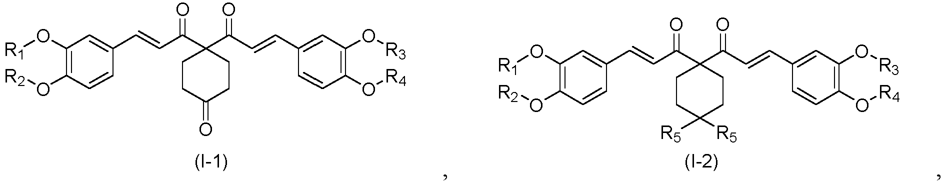 or 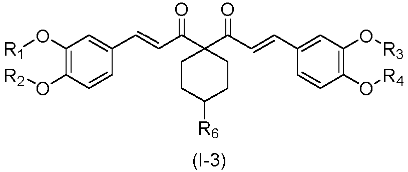 wherein R₁, R₂, R₃, and R₄ have the definitions as described above;
R₅ is halogen; R₆ is hydroxyl, amino, and the following groups that are unsubstituted or optionally substituted with one, two or more Rc: C₁₋₁₂ alkyl, C₁₋₁₂ alkoxy, C₃₋₂₀ cycloalkyl, 3- to 20-membered heterocyclyl, -NHC₁₋₁₂ alkyl, -N(C₁₋₁₂ alkyl)₂, -NHCOC₁₋₁₂ alkyl, -CONHC₁₋₁₂ alkyl, -NHC₃₋₂₀ cycloalkyl, -S(O)₂C₁₋₁₂ alkyl, and -COOC₁₋₁₂ alkyl.

In some embodiments of the present disclosure, 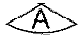 represents cyclohexyl substituted with =O, halogen, 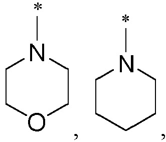 or -N(C₂H₅)₂.

In one embodiment of the present disclosure, 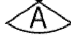 represents cyclohexyl substituted with =O or halogen, such as

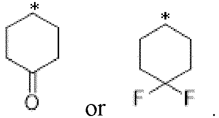

In some preferred embodiments of the present disclosure, the compound represented by formula I is selected from the following:

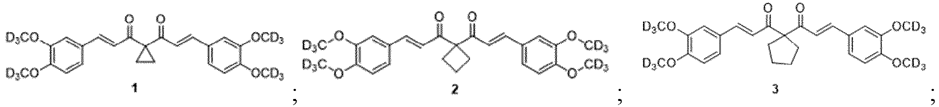

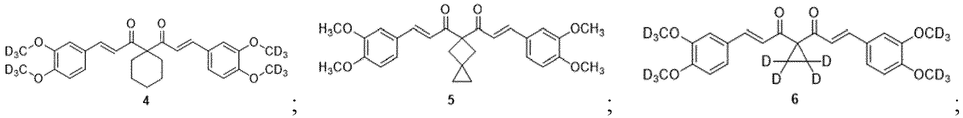

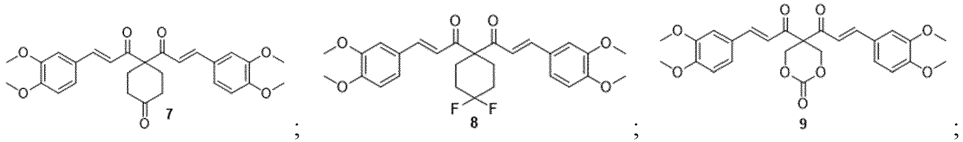

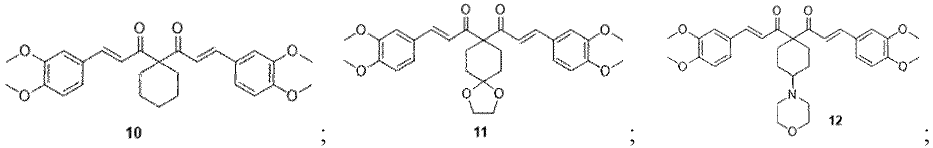

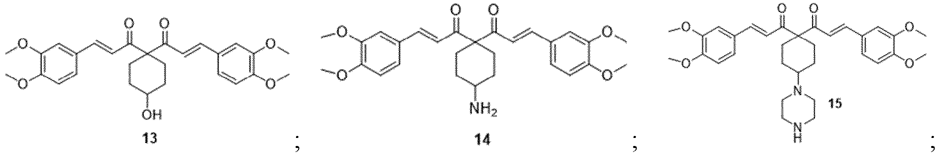

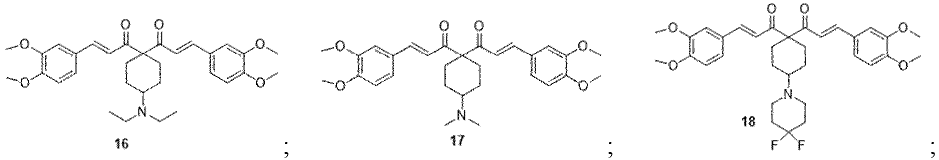

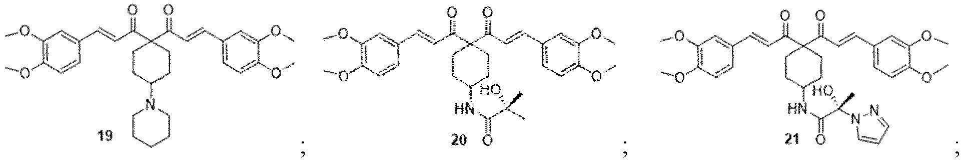

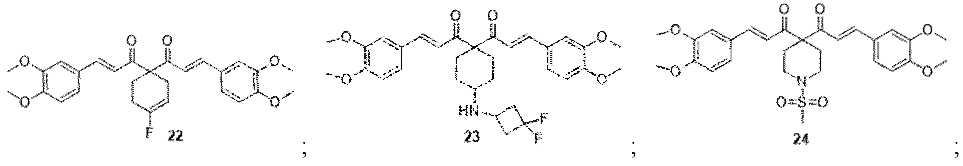

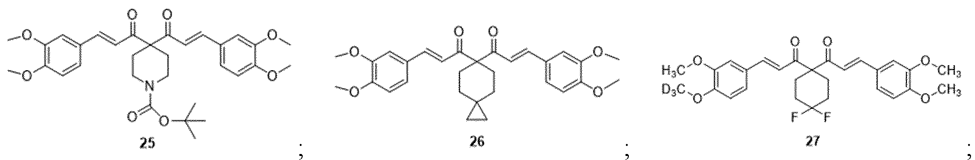

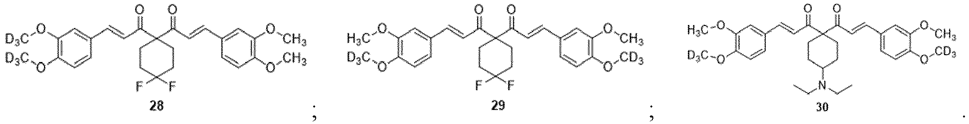

In some specific embodiments of the present disclosure, the compound represented by formula I is selected from the following:

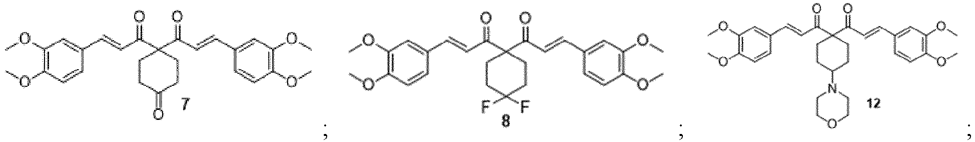

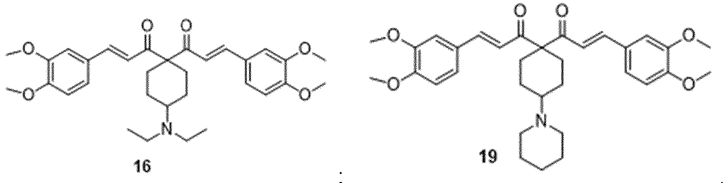

In some preferred embodiments of the present disclosure, the compound represented by formula I is selected from the following:

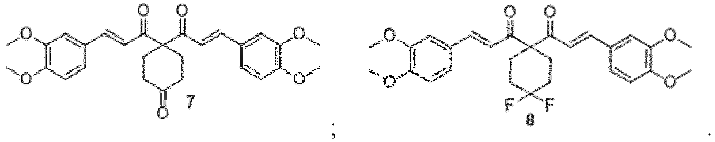

If the compounds of the present disclosure may be present in tautomeric forms, the present disclosure includes all tautomeric forms.

The compounds of the present disclosure may be present in stereoisomeric forms (enantiomers or diastereoisomers). Thus, the present disclosure includes enantiomers or diastereoisomers and their respective mixtures. Stereoisomerically homogeneous components can be separated in a known manner from such mixtures of enantiomers and/or diastereoisomers.

In some embodiments of the present disclosure, the pharmaceutically acceptable salt of the compound represented by formula I is hydrochloride thereof, e.g., the hydrochloride of compound 12, compound 14, compound 16, compound 18, compound 19, and compound 23.

The present disclosure further provides a preparation method for the compound represented by formula I described above, comprising the following steps: 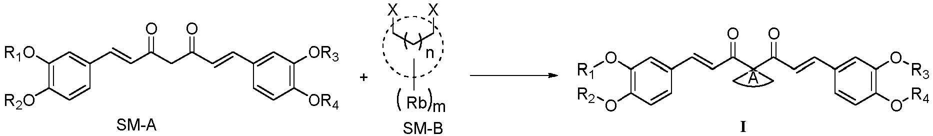 wherein R₁-R₄ and Rb have the definitions as described above, n is an integer from 0 to 17, m is an integer greater than or equal to 0, and X is halogen such as bromine; a compound represented by formula SM-A is reacted with a compound represented by formula SM-B to give the compound represented by formula I. Further, in the compound represented by formula I, different substituents may be introduced into ring A of the compound represented by formula I by conventional chemical synthesis methods. For example, some compounds represented by formula I may be prepared by the following methods, including:
reacting a compound represented by formula (I-1) with R₅-L to give a compound represented by formula (I-2), 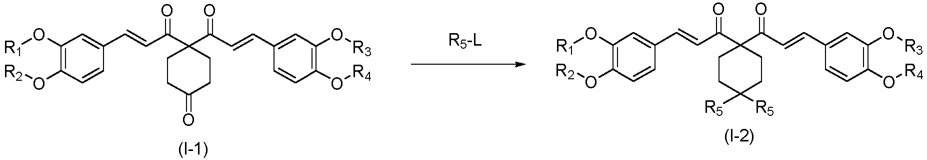
wherein R₁, R₂, R₃, R₄, and R₅ have the definitions as described above; L is a leaving group;
or reacting the compound represented by formula (I-1) with R₆-L to give a compound represented by formula (I-3), 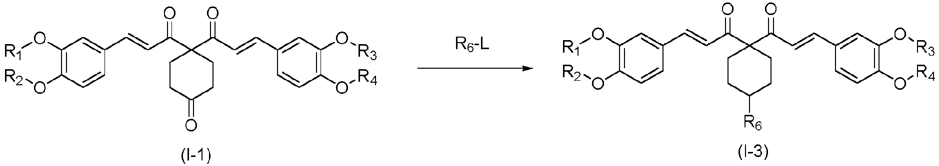
wherein R₁, R₂, R₃, R₄, and R₆ have the definitions as described above; L is a leaving group.

In some embodiments, n is an integer from 0 to 9, e.g., 0, 1, 2, 3, 4, or 5.

In some embodiments, m is an integer from 0 to 10, e.g., an integer from 0 to 5, such as 0, 1, 2, 3, or 4. The present disclosure further provides a pharmaceutical composition, comprising at least one of the compound represented by formula I, the racemate thereof, the stereoisomer thereof, the tautomer thereof, the solvate thereof, the polymorph thereof or the pharmaceutically acceptable salt thereof. According to the present disclosure, the pharmaceutical composition further comprises one or more pharmaceutically acceptable auxiliary materials.

Suitable routes of administration for the pharmaceutical composition of the present disclosure include, but are not limited to, oral, rectal, topical, buccal, parenteral, intramuscular, intradermal, intravenous, and transdermal administration.

According to the present disclosure, the pharmaceutical composition is for oral administration, and the pharmaceutical composition may be a tablet, a pill, a lozenge, a dragee, a capsule, or the like. According to the present disclosure, the pharmaceutical composition is for topical administration, and the pharmaceutical composition may be an ointment, a cream, a paste, a tincture, a plaster, a gel, a film, a paint, an aerosol, a spray, a foam, a microsponge, or the like.

The pharmaceutical composition of the present disclosure may be prepared by methods well known in the art, such as conventional methods of mixing, granulating, dragee-manufacturing, grinding, melting, emulsifying, dissolving, and the like. For example, a solid oral composition may be prepared by conventional mixing, filling, or tableting; a topical preparation may be prepared by conventional dissolving, mixing, and stirring. For example, it may be obtained by the following method: mixing an active compound with a solid auxiliary material, optionally grinding the resulting mixture, adding other suitable auxiliary materials if desired, and processing the mixture into granules as the cores of tablets or dragees. Suitable auxiliary materials include, but are not limited to, adhesives, diluents, disintegrants, lubricants, glidants, sweeteners, flavoring agents, etc. For example, a cream may be prepared by emulsifying, and may be prepared by the following method: heating and melting a greasy component and an oil-soluble component to give an oily phase, dissolving a water-soluble component in water and heating to give an aqueous phase, adding the aqueous phase into the oily phase, and stirring while adding until it condenses to give an ointment. Suitable auxiliary materials include, but are not limited to, matrixes, pH regulators, and transdermal absorption enhancers.

The present disclosure provides use of the compound represented by formula I, the racemate thereof, the stereoisomer thereof, the tautomer thereof, the solvate thereof, the polymorph thereof or the pharmaceutically acceptable salt thereof described above, or the pharmaceutical composition of the present disclosure for manufacturing a medicament for the treatment, prevention, or amelioration of symptoms or diseases of androgen-related disorders.

According to the present disclosure, non-limiting examples of the symptoms or diseases of androgen-related disorders are: androgen-related inflammation, including wounds (the compounds of the present disclosure can aid in wound healing), acne, atopic dermatitis, rheumatoid arthritis, psoriasis, and rosacea; Kennedy's disease (spinal and bulbar muscular atrophy or SBMA), and polyglutamine-mediated degeneration of motor neurons; androgen-related cancers such as prostate cancer, bladder cancer, breast cancer, ovarian cancer, endometrial cancer, hepatocellular carcinoma, hepatocellular carcinoma, central nervous system cancer, skin cancer, lymphoma, leukemia, esophageal cancer, gastric cancer, colon cancer, and pancreatic cancer; alopecia, including androgenetic alopecia; comedone; and hirsutism.

The present disclosure further provides a method for treating, preventing, or ameliorating symptoms or diseases of androgen-related disorders, comprising administering to an individual in need thereof a prophylactically or therapeutically effective amount of the compound represented by formula I, the racemate thereof, the stereoisomer thereof, the tautomer thereof, the solvate thereof, the polymorph thereof or the pharmaceutically acceptable salt thereof of the present disclosure, or the pharmaceutical composition of the present disclosure.

According to the present disclosure, non-limiting examples of the symptoms or diseases of androgen-related disorders are: androgen-related inflammation, including wounds (the compounds of the present disclosure can aid in wound healing), acne, atopic dermatitis, rheumatoid arthritis, psoriasis, and rosacea; polyglutamine-mediated degeneration of motor neurons, and Kennedy's disease (spinal and bulbar muscular atrophy or SBMA); androgen-related cancers such as prostate cancer, bladder cancer, breast cancer, ovarian cancer, endometrial cancer, hepatocellular carcinoma, hepatocellular carcinoma, central nervous system cancer, skin cancer, lymphoma, leukemia, esophageal cancer, gastric cancer, colon cancer, and pancreatic cancer; alopecia, including androgenetic alopecia; comedone; and hirsutism.

In all of the administration methods of the compound represented by formula I described herein, the daily dose administered is from 0.01 to 200 mg/kg body weight.

According to the present disclosure, the administration regimen may be adjusted to provide the optimum desired response. For example, a single oral dose may be administered, several separate doses may be administered over time, or the dose may be proportionally decreased or increased as indicated by the exigencies of the treatment situation. It is noted that dose values may vary with the type and severity of the condition to be alleviated, and may include single or multiple doses. It is further understood that for any particular individual, the specific dosage regimen will be adjusted over time according to the individual need and the professional judgment of the person administering the composition or supervising the administration of the composition.

### Definitions and Description

Unless otherwise stated, all technical and scientific terms used herein have the same meaning as commonly understood by those skilled in the art to which the present disclosure belongs. All patents and publications referred to herein are incorporated herein by reference in their entirety.

Unless otherwise stated, the following definitions as used herein should be applied. When referring to a trade name, it is intended to refer to its corresponding commercial product or its active ingredient. As used herein, the terms "include", "comprise", "have", "contain", or "involve", and other variants thereof herein, are inclusive or open-ended and do not exclude other unrecited elements or method steps.

Herein, the term "optional" or "optionally" indicates both the presence and absence of the stated feature, which means that the subsequently described event may, but does not necessarily, occur, thus including both instances where the event occurs or does not occur. For example, "heterocyclyl group optionally substituted with alkyl" means that the alkyl may be, but not necessarily, present, thus including instances where the heterocyclyl group is substituted with alkyl and the heterocyclyl group is not substituted with alkyl.

Herein, the term "halogen" refers to fluorine, chlorine, bromine, and/or iodine. Accordingly, the term "halo" refers to fluoro, chloro, bromo, and/or iodo. Within the scope of the present disclosure, when an atom, a residue, a group, or a moiety is halogenated, the atom at the halogenated position may be monosubstituted, disubstituted, or polysubstituted with halogen atom(s) up to full substitution.

In the present application, "*" refers to a connection site.

The term "C₁₋₁₂ alkyl" should be understood to refer to a linear or branched saturated monovalent hydrocarbyl group having 1-12 carbon atoms, preferably C₁₋₆ alkyl. "C₁₋₆ alkyl" should be understood to preferably refer to a linear or branched saturated monovalent hydrocarbyl group having 1, 2, 3, 4, 5, or 6 carbon atoms. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or an isomer thereof. Particularly, the group has 1, 2, or 3 carbon atoms ("C₁₋₃ alkyl"), such as methyl, ethyl, n-propyl, or isopropyl.

The term "C₃₋₂₀ cycloalkyl" should be understood to refer to a saturated monovalent monocyclic or bicyclic hydrocarbon ring having 3-20 carbon atoms, preferably "C₃₋₁₂ cycloalkyl". The term "C₃₋₁₂ cycloalkyl" should be understood to refer to a saturated monovalent monocyclic or bicyclic hydrocarbon ring having 3, 4, 5, 6, 7, 8, 9, 10, 11, or 12 carbon atoms. The C₃₋₁₂ cycloalkyl may be a monocyclic hydrocarbon group, such as cyclopropyl, cyclobutyl, cyclopentyl, cyclohexyl, cycloheptyl, cyclooctyl, cyclononyl or cyclodecyl, or a bicyclic hydrocarbon group, such as a decaline ring.

The term "C₄₋₂₀ cycloalkenyl" should be understood to refer to an unsaturated monovalent monocyclic or bicyclic hydrocarbon ring having 4 to 20 carbon atoms and containing at least 1 unsaturated double bond, such as 1, 2, or 3 unsaturated double bonds, preferably "C₄₋₁₂ cycloalkenyl". The term "C₄₋₁₂ cycloalkenyl" should be understood to refer to an unsaturated monovalent monocyclic or bicyclic hydrocarbon ring having 4, 5, 6, 7, 8, 9, 10, 11, or 12 carbon atoms and 1, 2, or 3 unsaturated double bonds. The C₄₋₁₂ cycloalkyl is, for example, cyclohexenyl.

The term "3- to 20-membered heterocyclyl" refers to a saturated monovalent monocyclic or bicyclic hydrocarbon ring containing 1-5 heteroatoms independently selected from N, O, and S, preferably "3- to 12-membered heterocyclyl". The term "3- to 12-membered heterocyclyl" refers to a saturated monovalent monocyclic or bicyclic hydrocarbon ring containing 1-5, preferably 1-3, heteroatoms selected from N, O, and S. The heterocyclyl may be attached to the rest of the molecule through any one of the carbon atoms or the nitrogen atom (if present). In particular, the heterocyclyl may include, but is not limited to, 4-membered rings such as azetidinyl and oxetanyl; 5-membered rings such as tetrahydrofuranyl, dioxolyl, pyrrolidinyl, imidazolidinyl, pyrazolidinyl and pyrrolinyl; 6-membered rings such as tetrahydropyranyl, piperidyl, morpholinyl, dithianyl, thiomorpholinyl, piperazinyl and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[*c*]pyrrol-2(1*H*)-yl ring, or a 5,6-membered bicyclic ring such as a hexahydropyrrolo[1,2-*a*]pyrazin-2(1*H*)-yl ring. The ring containing the nitrogen atom may be partially unsaturated, i.e., it may contain one or more double bonds, for example, but not limited to, 2,5-dihydro-1*H*-pyrrolyl, 4*H*-[1,3,4]thiadiazinyl, 4,5-dihydrooxazolyl, or 4*H*-[1,4]thiazinyl, or it may be benzo-fused, for example, but not limited to, dihydroisoquinolyl. According to the present disclosure, the heterocyclyl is non-aromatic.

The C₃₋₂₀ cycloalkyl in the term "spiro ring formed from C₃₋₂₀ cycloalkyl and C₃₋₂₀ cycloalkyl" is as defined above, and the term refers to a spiro ring formed from two C₃₋₂₀ cycloalkyl groups by sharing 1 carbon atom.

The C₃₋₂₀ cycloalkyl and 3- to 20-membered heterocyclyl in the term "spiro ring formed from C₃₋₂₀ cycloalkyl and 3- to 20-membered heterocyclyl" are as defined above, and the term refers to a spiro ring formed from one C₃₋₂₀ cycloalkyl group and one 3- to 20-membered heterocyclyl group by sharing 1 carbon atom.

The 3- to 20-membered heterocyclyl in the term "spiro ring formed from 3- to 20-membered heterocyclyl and 3- to 20-membered heterocyclyl" is as defined above, and the term refers to a spiro ring formed from two 3- to 20-membered heterocyclyl groups by sharing 1 carbon atom.

The term "5- to 20-membered heteroaryl" should be understood to refer to a monovalent aromatic monocyclic, bicyclic or tricyclic ring system that has 5-20 ring atoms and contains 1-5 heteroatoms independently selected from N, O, and S, such as "5- to 12-membered heteroaryl". The term "5- to 12-membered heteroaryl" should be understood to refer to a monovalent aromatic monocyclic, bicyclic or tricyclic ring system that has 5, 6, 7, 8, 9, 10, 11, or 12 ring atoms, in particular 5, or 6, or 9, or 10 carbon atoms, contains 1-5, preferably 1-3, heteroatoms independently selected from N, O, and S, and may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4*H*-pyrazolyl and the like and benzo derivatives thereof, such as benzofuranyl, benzothienyl, benzoxazolyl, benzoisoxazolyl, benzimidazolyl, benzotriazolyl, indazolyl, indolyl, isoindolyl and the like; or pyridinyl, pyridazinyl, pyrimidinyl, pyrazinyl, triazinyl and the like and benzo derivatives thereof, such as quinolyl, quinazolinyl, isoquinolyl and the like; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like.

The term "preventing or treating" means administering the compound or preparation described herein to prevent, relieve or eliminate a disease or one or more symptoms associated with the disease, and includes:
(i) preventing the occurrence of a disease or a disease state in a mammal, particularly when the mammal is predisposed to the disease state but has not yet been diagnosed as having the disease state;
(ii) inhibiting the disease or the disease state, i.e., arresting its development; and
(iii) alleviating the disease or the disease state, i.e., causing regression of the disease or the disease state.

The term "therapeutically effective amount" refers to an amount of the compound of the present disclosure that (i) treats or prevents a particular disease, condition, or disorder, (ii) reduces, relieves, or eliminates one or more symptoms of the particular disease, condition, or disorder, or (iii) prevents or delays the onset of one or more symptoms of the particular disease, condition, or disorder described herein. The amount of the compound of the present disclosure that constitutes a "therapeutically effective amount" varies depending on the compound, the disease state and its severity, the route of administration, and the age of the mammal to be treated, but may be routinely determined by those skilled in the art in view of their own knowledge and this disclosure.

The term "pharmaceutically acceptable" refers to those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The pharmaceutically acceptable salt of the compound of the present disclosure includes salts thereof that are formed with pharmaceutically acceptable acids and salts thereof that are formed with pharmaceutically acceptable bases.

The term "pharmaceutically acceptable acids" as used herein refers to, for example, hydrochloric acid, hydrobromic acid, sulfuric acid, nitric acid, phosphoric acid, carbonic acid, formic acid, acetic acid, acetoacetic acid, trifluoroacetic acid, propionic acid, pyruvic acid, butyric acid, caproic acid, heptanoic acid, undecanoic acid, lauric acid, stearic acid, palmitic acid, oxalic acid, methanesulfonic acid, trifluoromethanesulfonic acid, ethanedisulfonic acid, isethionic acid, 1,5-naphthalenedisulfonic acid, 2-naphthalenesulfonic acid, camphorsulfonic acid, sulfamic acid, lactic acid, benzenesulfonic acid, p-toluenesulfonic acid, malonic acid, succinic acid, glutaric acid, adipic acid, maleic acid, fumaric acid, lactic acid, tartaric acid, citric acid, malic acid, benzoic acid, salicylic acid, cinnamic acid, naphthoic acid, pamoic acid, nicotinic acid, orotic acid, methylsulfuric acid, dodecylsulfuric acid, glutamic acid, aspartic acid, gluconic acid, glucuronic acid, or any combination thereof.

The term "pharmaceutically acceptable bases" as used herein refers to, for example, inorganic bases (alkali metal hydroxides or alkaline earth metal hydroxides, etc.) or organic bases (e.g., amines (primary, secondary or tertiary), etc.). Examples of suitable salts include, but are not limited to, organic salts obtained from amino acids, ammonia, primary amines, secondary amines, tertiary amines, and cyclic amines (e.g., diethylamine salts, piperidine salts, morpholine salts, piperazine salts, choline salts, meglumine salts, tromethamine salts, etc.), and inorganic salts obtained from sodium, calcium, potassium, magnesium, manganese, iron, copper, zinc, aluminum, and lithium. The term "solvate" refers to a complex formed by coordination of the compound of the present disclosure in the solid or liquid state with solvent molecules. Hydrate is a particular form of solvate in which coordination occurs with water.

The term "pharmaceutical composition" refers to a mixture consisting of one or more of the compounds of the present disclosure or salts thereof and pharmaceutically acceptable auxiliary materials. The pharmaceutical composition is intended to facilitate the administration of the compound of the present disclosure to an organic entity.

The compound of the present disclosure is formulated into tablets, pills, lozenges, dragees, capsules, and the like, for oral administration to patients. The compound of the present disclosure may also be formulated into ointments, creams, pastes, tinctures, plasters, gels, films, paints, aerosols, sprays, foams, microsponges, and the like, for topical administration to patients.

The term "pharmaceutically acceptable auxiliary materials" refers to those auxiliary materials which do not have a significant irritating effect on organisms and do not impair the bioactivity and performance of the active compound. Suitable auxiliary materials are well known to those skilled in the art, for example, carbohydrates, waxes, water-soluble and/or water-expandable polymers, hydrophilic or hydrophobic materials, gelatin, oils, solvents, water, and the like.

The pharmaceutical composition of the present application may be prepared by combining the compound of the present application with a suitable pharmaceutically acceptable auxiliary material, and may be formulated, for example, into a solid, semisolid, liquid, or gaseous preparation such as tablet, pill, capsule, powder, granule, ointment, emulsion, suspension, suppository, injection, inhalant, gel, microsphere, aerosol, and the like.

Typical routes of administration of the compound or the pharmaceutically acceptable salt thereof or the pharmaceutical composition thereof of the present application include, but are not limited to, oral, rectal, topical, inhalation, parenteral, sublingual, intravaginal, intranasal, intraocular, intraperitoneal, intramuscular, subcutaneous, and intravenous administration. The oral administration has better patient compliance than the intravenous administration.

The pharmaceutical composition of the present application may be manufactured by methods well known in the art, such as conventional methods of mixing, dissolving, granulating, dragee-manufacturing, levigating, emulsifying, lyophilizing, and the like.

In some embodiments, the pharmaceutical composition is in an oral form. For oral administration, the pharmaceutical composition may be formulated by mixing the active compounds with pharmaceutically acceptable auxiliary materials well known in the art. These auxiliary materials enable the compound of the present application to be formulated into tablets, pills, lozenges, dragees, capsules, liquids, gels, slurries, suspensions, and the like, for oral administration to patients. For example, a solid oral composition may be prepared by conventional mixing, filling, or tableting. For example, it may be obtained by the following method: mixing the active compound with a solid auxiliary material, optionally grinding the resulting mixture, adding other suitable auxiliary materials if desired, and processing the mixture into granules as the cores of tablets or dragees. Suitable auxiliary materials include, but are not limited to, adhesives, diluents, disintegrants, lubricants, glidants, sweeteners, flavoring agents, etc.

The pharmaceutical composition of the present disclosure may also be adapted for topical administration, such as creams, gels, foams, or tinctures in suitable unit dosage forms. A topical preparation may be prepared by conventional dissolving, mixing, and stirring. For example, a cream may be prepared by emulsifying, and may be prepared by the following method: heating and melting a greasy component and an oil-soluble component to give an oily phase, dissolving a water-soluble component in water and heating to give an aqueous phase, adding the aqueous phase into the oily phase, and stirring while adding until it condenses to give an ointment. Suitable auxiliary materials include, but are not limited to, matrixes, pH regulators, and transdermal absorption enhancers.

The term "androgen" refers to male hormones such as testosterone and dihydrotestosterone (DHT). DHT is a product of conversion of testosterone by 5-α-reductase. Androgens stimulate or control the development and maintenance of male characteristics and other physiological functions in vertebrates and activate or regulate genes by binding to androgen receptors, which in turn bind to androgen/AR-controlled genes (DNA).

The term "androgen receptor" or "AR" refers to an intracellular receptor that specifically binds to an androgen, including testosterone and dihydrotestosterone (DHT). AR includes all mammalian androgen receptor isomorphs, binding variants, and polymorphs.

The term "androgenetic alopecia" in humans refers to symptoms and conditions of alopecia related to androgen levels in the body. It is generally accepted that androgenetic alopecia is caused by the sensitivity of the hair follicles or surrounding tissues to androgens, which is a genetic factor and is often inherited in families. Androgenetic alopecia has been associated in men with several other medical conditions including coronary heart disease, enlargement of the prostate, prostate cancer, insulin resistance conditions (such as diabetes and obesity), and hypertension. In women, alopecia may be associated with an increased risk of polycystic ovary syndrome (PCOS). PCOS is characterized by a hormone imbalance that can lead to menstrual disorders, acne, excess body hair (hirsutism), and weight gain. Alopecia is often associated with androgen accumulation or increased amounts of androgens in women. Androgenetic alopecia is the most common form of alopecia in men, and such a condition is commonly referred to as male-type baldness. The hair is lost in a clearly discernible form, starting on the temples on both sides. Over time, the hairline recedes to form the typical "M" shape. The hair also thins at the top of the head, often developing a partial or complete baldness. In women, the appearance is that the hair becomes thinner throughout the head and the hairline does not recede. Androgenetic alopecia in women rarely leads to complete baldness.

The term "anti-AR activity" refers to the ability of the compound of the present disclosure to reduce AR expression in human prostate cancer cells LNCaP, and the ability of the compound of the present disclosure to reduce AR expression is expressed in AR reduction percentage (%) herein. More reduced AR expression indicates greater anti-AR activity of the compound, i.e., a greater value of AR reduction percentage (%) indicates greater anti-AR activity. The AR reduction percentage (%) is calculated as follows: AR reduction percentage (%) = (AR in DHT group /GAPDH value - AR in test compound group/GAPDH value)/AR in DHT group /GAPDH value × 100%.

As used herein, "individual" includes humans or non-human animals. Exemplary human individuals include human individuals with a disease (e.g., a disease described herein), which are referred to as patients, or normal individuals. In the present disclosure, "non-human animals" include all vertebrates, such as non-mammals (e.g., birds, amphibians, reptiles) and mammals, such as non-human primates, livestock and/or domesticated animals (e.g., sheep, dogs, cats, cows, pigs, etc.).

### Beneficial Effects

The present disclosure provides a curcumin derivative represented by formula I, which has good prostate tumor cell proliferation inhibitory activity and anti-AR activity. Moreover, the curcumin derivative of the present disclosure has good *in-vivo* metabolism properties, high AUC and Cₘₐₓ, and good druggability, and can be used for preventing and/or treating diseases of androgen-related disorders. In addition, the curcumin derivative of the present disclosure also has a good hair growth-promoting effect, and can effectively increase the number of hair follicles and the growth length of hair.

### BRIEF DESCRIPTION OF THE DRAWINGS

FIG. 1 shows Western blot images of reduced AR protein expression in LNCaP cells with different concentrations of the compounds of the present disclosure;
FIG. 2 shows pharmacokinetic curves of the compounds in the plasma of male rats after oral administration of the compounds of the present disclosure;
FIG. 3 shows photographs of hair growth and representative HE staining pathological images of skin tissues on day 18 of the test in Test Example 4 for various groups of mice in the hair growth promotion test.

### DETAILED DESCRIPTION

The general-formula compounds of the present disclosure, the preparation method therefor, and use thereof are described in detail with reference to the following specific examples. It should be understood that the following examples are merely exemplary illustration and explanation of the present disclosure and should not be construed as limiting the protection scope of the present disclosure. All techniques implemented based on the content described above of the present disclosure are encompassed within the protection scope of the present disclosure.

The intermediate compounds of the present disclosure can be prepared by a variety of synthetic methods well known to those skilled in the art, including the specific embodiments listed below, embodiments formed by combinations thereof with other chemical synthetic methods, and equivalent substitutions thereof known to those skilled in the art. Preferred embodiments include but are not limited to the examples of the present disclosure.

The chemical reactions of the specific embodiments of the present disclosure are carried out in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction scheme based on the existing embodiments.

The present disclosure is described in detail below through examples; however, these examples are not intended to limit the present disclosure in any way.

Experimental methods without specified conditions in the following examples are generally conducted under conventional conditions, or conditions recommended by the manufacturer. Unless otherwise indicated, the percentages and the number of parts are calculated by weight, and the starting materials and reagents used in the following examples are commercially available or may be manufactured by known methods.

The following abbreviations are used in the present disclosure: DHT for dihydrotestosterone; clascoterone (purchased from Nanjing Chemlin Chemical Industry Co., Ltd.), which is a chemical drug on the market, the target indications including androgenetic alopecia, acne, and the like, and the mechanism of action thereof being as follows: competitively inhibiting the binding of DHT to AR, thereby achieving an anti-androgen effect; dimethylcurcumin (purchased from Nanjing Chemlin Chemical Industry Co., Ltd.); starting material A having a structural formula of 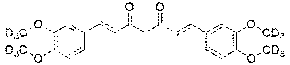 (purchased from Nanjing Chemlin Chemical Industry Co., Ltd.); starting material B having a structural formula of 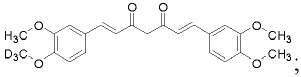 starting material C having a structural formula of 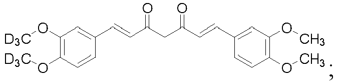 and starting material D having a structural formula of 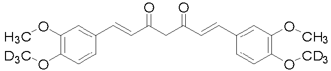 (starting materials B to D are all purchased from Nanjing Jiming Biotechnology Co., Ltd.).

### Example 1. Synthesis of Compound 1

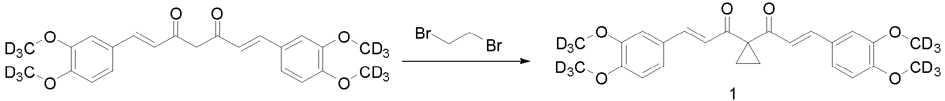

Starting material A (408 mg, 1.00 mmol, 1.0 eq), 1,2-dibromoethane (225 mg, 1.20 mmol), Cs₂CO₃ (814 mg, 2.5 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phases were combined and washed with saturated brine to remove DMF. The resulting solution was dried over anhydrous sodium sulfate, concentrated, and subjected to column chromatography (petroleum ether/ethyl acetate 10:1-2:1) to give a light yellow solid (39 mg).

¹H NMR (400 MHz, DMSO-*d₆*) δ 7.53 (d, J = 15.8 Hz, 2H), 7.30 (d, J = 2.0 Hz, 2H), 7.24 (dd, J = 8.4, 2.1 Hz, 2H), 6.97 (d, J = 8.3 Hz, 2H), 6.94 (d, J = 15.7 Hz, 2H), 1.54 (brs, 4H). MS m/z: 435.14 [M+H]⁺.

### Example 2. Synthesis of Compound 2

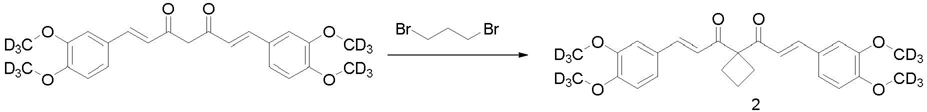

Starting material A (408 mg, 1.00 mmol, 1.0 eq), 1,3-dibromopropane (242 mg, 1.20 mmol), Cs₂CO₃ (814 mg, 2.5 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phases were combined and washed with saturated brine to remove DMF. The resulting solution was dried over anhydrous sodium sulfate, concentrated, and subjected to column chromatography (petroleum ether/ethyl acetate 10:1-2:1) to give a light yellow solid (90 mg).

¹H NMR (400 MHz,CDCl₃) δ 7.57 (d, J = 15.6 Hz, 1H), 7.33 (d, J = 15.8 Hz, 1H), 7.20 - 6.91 (m, 6H), 6.84 (d, J = 8.2 Hz, 1H), 6.79 (d, J = 8.2 Hz, 1H), 4.36 - 4.06 (m, 2H), 2.76 - 2.46 (m, 2H), 2.12 - 1.83 (m, 2H). MS m/z: 449.22 [M+H]⁺.

### Example 3. Synthesis of Compound 3

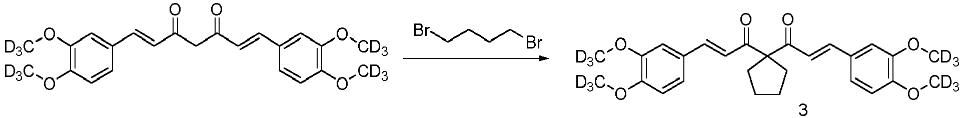

Starting material A (408 mg, 1.00 mmol, 1.0 eq), 1,4-dibromobutane (260 mg, 1.20 mmol), Cs₂CO₃ (814 mg, 2.5 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phases were combined and washed with saturated brine to remove DMF. The resulting solution was dried over anhydrous sodium sulfate, concentrated, and subjected to column chromatography (petroleum ether/ethyl acetate 10:1-2:1) to give a light yellow solid (87 mg).

¹H NMR (400 MHz, DMSO- *d₆*) δ 7.55 (d, J = 15.6 Hz, 2H), 7.32 - 7.18 (m, 4H), 6.96 (d, J = 8.3 Hz, 2H), 6.81 (d, J = 15.7 Hz, 2H), 2.31 - 2.15 (m, 4H), 1.67 - 1.50 (m, 4H). MS m/z: 463.20 [M+H]⁺.

### Example 4. Synthesis of Compound 4

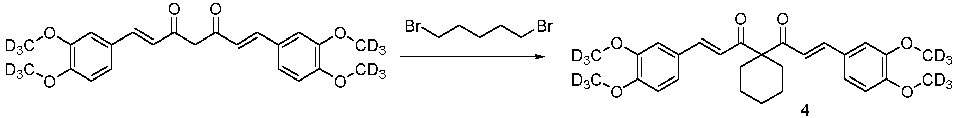

Starting material A (408 mg, 1.00 mmol, 1.0 eq), 1,4-dibromopentane (277 mg, 1.20 mmol), Cs₂CO₃ (814 mg, 2.5 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phases were combined and washed with saturated brine to remove DMF. The resulting solution was dried over anhydrous sodium sulfate, concentrated, and subjected to column chromatography (petroleum ether/ethyl acetate 10:1-2:1) to give a light yellow solid (92 mg).

¹H NMR (400 MHz, DMSO- *d₆*) δ 7.56 (d, J = 15.5 Hz, 2H), 7.37 - 7.18 (m, 4H), 7.03 (d, J = 15.5 Hz, 2H), 6.96 (d, J = 8.3 Hz, 2H), 2.21 - 1.96 (m, 4H), 1.60 - 1.28 (m, 6H). MS m/z: 477.23 [M+H]⁺.

### Example 5. Synthesis of Compound 5

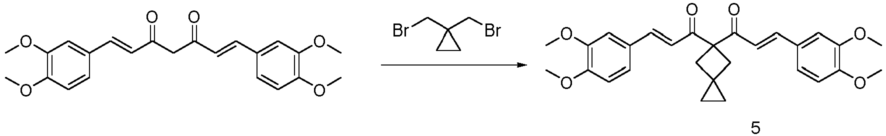

Dimethylcurcumin (500 mg, 1.26 mmol, 1.0 eq), 1,1-bis-(bromomethyl)cyclopropane (340 mg, 1.50 mmol), Cs₂CO₃ (1.00 g, 3.11 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phases were combined and washed with saturated brine to remove DMF. The resulting solution was dried over anhydrous sodium sulfate, concentrated, and subjected to column chromatography (petroleum ether/ethyl acetate 10:1-2:1) to give a light yellow solid (20 mg).

¹H NMR (400 MHz, CDCl₃) δ 8.20 (d, J = 15.8 Hz, 1H), 7.56 (d, J = 15.6 Hz, 1H), 7.33 (d, J = 16.1 Hz, 1H), 7.24 - 6.79 (m, 6H), 6.72 (d, J = 15.5 Hz, 1H), 3.92 (s, 12H), 1.63 (brs, 4H), 0.70 (brs, 4H). MS m/z: 463.16 [M+H]⁺.

### Example 6. Synthesis of Compound 6

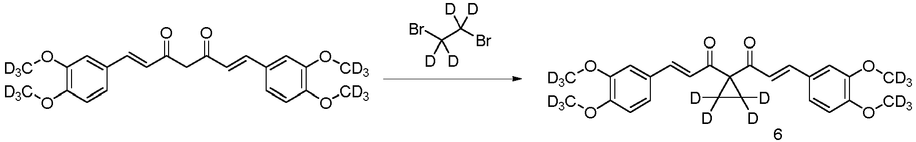

Starting material A (408 mg, 1.00 mmol, 1.0 eq), deuterated dibromoethane (230 mg, 1.20 mmol), Cs₂CO₃ (814 mg, 2.5 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phases were combined and washed with saturated brine to remove DMF. The resulting solution was dried over anhydrous sodium sulfate, concentrated, and subjected to column chromatography (petroleum ether/ethyl acetate 10:1-2:1) to give a light yellow solid (30 mg).

¹H NMR (400 MHz, CDCl₃) δ 7.62 (d, J = 15.8 Hz, 2H), 7.12 (dd, J = 8.3, 2.1 Hz, 2H), 7.00 (d, J = 2.0 Hz, 2H), 6.83 (d, J = 8.3 Hz, 2H), 6.79 (d, J = 15.7 Hz, 2H). MS m/z: 439.18 [M+H]⁺.

### Example 7. Synthesis of Compound 7

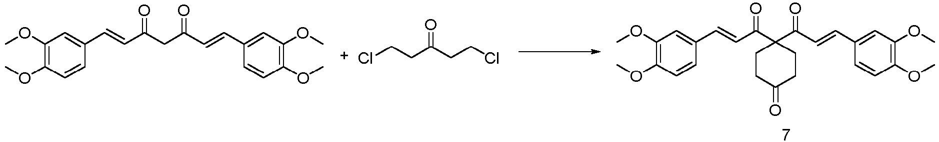

Dimethylcurcumin (10.0 g, 25.22 mmol, 1.0 eq), 1,5-dichloropentanone (3.91 g, 25.22 mmol), KBr (12.01 g, 100.9 mmol), K₂CO₃ (10.46 g, 75.67 mmol), and DMF (300 mL) were added to a reaction flask, and the resulting mixture was stirred at 50 °C for 20 h. After completion of the reaction, water (700 mL) and ethyl acetate (400 mL) were added. Liquid separation was performed. The aqueous phase was extracted with ethyl acetate (400 mL). The organic phases were combined and washed with saturated brine to remove DMF. The resulting solution was dried over anhydrous sodium sulfate, concentrated, and subjected to column chromatography (petroleum ether/ethyl acetate 10:1-2:1) to give a yellow foamy solid (3.6 g).

¹H NMR (400 MHz, CDCl₃) 7.75 (d, J = 15.5 Hz, 2H), 7.15 (dd, J = 8.4, 2.0 Hz, 2H), 7.02 (d, J = 2.0 Hz, 2H), 6.85 (d, J = 8.4 Hz, 2H), 6.74 (d, J = 15.4 Hz, 2H), 3.91 (s, 12H), 2.56 - 2.40 (m, 8H). MS m/z: 479.29 [M+H]⁺.

### Example 8. Synthesis of Compound 8

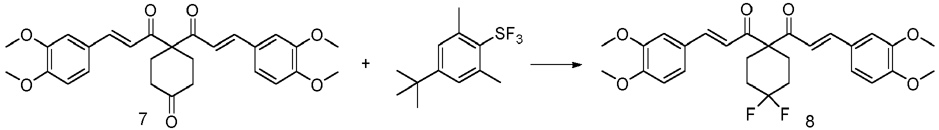

Under nitrogen atmosphere, compound 7 (2.0 g, 4.18 mmol), dichloromethane (20 mL), and 1 drop of pyridine hydrofluoride were added to a reaction flask. The resulting mixture was cooled to 0 °C under stirring, and 4-*tert*-butyl-2,6-dimethylphenylsulfur trifluoride (2.1 g, 8.36 mmol) was added in one portion. The mixture was reacted at 10-15 °C for 6 h. The reaction liquid was washed with water, dried over anhydrous sodium sulfate, and subjected to column chromatography (petroleum ether/ethyl acetate 10:1-2:1) to give a crude product, which was subjected to preparative HPLC (acetonitrile/water 10:90-80:20) to give a light yellow solid (150 mg).

¹H NMR (400 MHz, CDCl₃) δ 7.71 (d, J = 15.4 Hz, 2H), 7.14 (d, J = 8.3 Hz, 2H), 7.09 - 6.96 (m, 2H), 6.85 (d, J = 8.4 Hz, 2H), 6.72 (d, J = 15.4 Hz, 2H), 3.91 (s, 12H), 2.45 - 2.18 (m, 4H), 2.12 - 1.91 (m, 4H). ¹⁹F NMR (376 MHz, CDCl₃) δ -97.28. MS m/z: 501.27 [M+H]⁺.

### Example 9. Synthesis of Compound 9

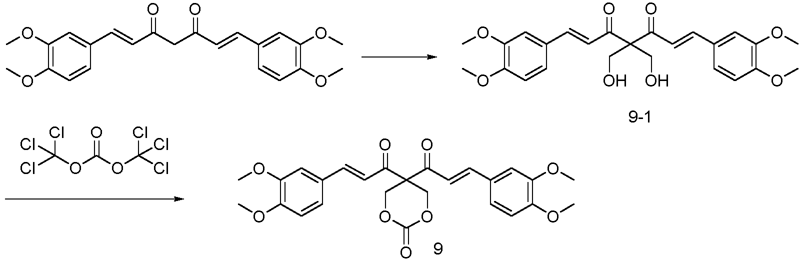

### Step (1): synthesis of intermediate 9-1

Dimethylcurcumin (2.00 g, 5.0 mmol) and tetrahydrofuran (16 mL) were successively added to a reaction flask. The resulting mixture was stirred and dissolved. The reaction temperature of the mixture was controlled to be -1 °C to 0 °C, and an aqueous formaldehyde solution (37-40%, 860 mg, 10.6 mmol) and a catalytic amount of 1,8-diazabicyclo[5.4.0]undec-7-ene (93 mg) were successively added into the mixture. The mixture was then reacted at 0-10 °C for 1-2 h, concentrated by rotary evaporation at room temperature, and subjected to column chromatography (MeOH/CH₂Cl₂ 1:100-1:50) to give a yellow solid (900 mg).

¹H NMR (400 MHz, CDCl₃) δ 7.73 (d, J = 15.4 Hz, 2H), 7.15 (dd, J = 8.3, 2.1 Hz, 2H), 7.01 (d, J = 2.1 Hz, 2H), 6.84 (d, J = 8.4 Hz, 2H), 6.73 (d, J = 15.4 Hz, 2H), 4.34 (d, J = 8.0 Hz, 4H), 3.90 (s, 12H), 2.97 (t, J = 8.0 Hz, 2H).

### Step (2): synthesis of compound 9

Intermediate 9-1 (300 mg, 0.65 mmol), dichloromethane (7 mL), and pyridine (156 mg, 1.97 mmol) were added to a reaction flask under nitrogen atmosphere, and the resulting mixture was cooled to - 70 °C under stirring. A solution of bis(trichloromethyl)carbonate (97 mg, 0.33 mmol) in dichloromethane (1 mL) was added dropwise into the mixture, and the mixture was reacted at -70 °C for 2 h. The reaction liquid was washed with water, dried over anhydrous sodium sulfate, and subjected to column chromatography (petroleum ether/ethyl acetate 10:1-2:1) to give a light yellow solid (210 mg). MS m/z: 483.35 [M+H]⁺.

### Example 10. Synthesis of Compound 10

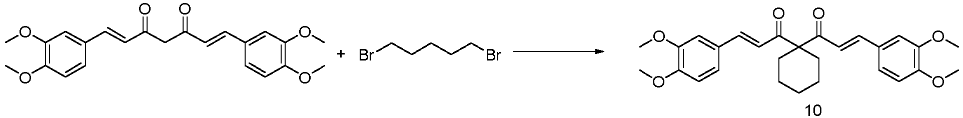

Dimethylcurcumin (500 mg, 1.26 mmol, 1.0 eq), 1,5-dibromopentane (435 mg, 1.89 mmol), K₂CO₃ (523 mg, 3.78 mmol), and DMF (15 mL) were added to a reaction flask, and the resulting mixture was stirred at 50 °C for 20 h. After completion of the reaction, water (50 mL) and ethyl acetate (20 mL) were added into the mixture. Liquid separation was performed. The aqueous phase was extracted with ethyl acetate (20 mL). The organic phases were combined and washed with saturated brine to remove DMF. The resulting solution was dried over anhydrous sodium sulfate, concentrated, and subjected to column chromatography (petroleum ether/ethyl acetate 10:1-2:1) to give a yellow foamy solid (150 mg).

¹H NMR (400 MHz, CDCl₃) δ 7.66 (d, J = 15.5 Hz, 2H), 7.12 (dd, J = 8.3, 1.9 Hz, 2H), 7.01 (d, J = 2.0 Hz, 2H), 6.83 (d, J = 8.3 Hz, 2H), 6.75 (d, J = 15.4 Hz, 2H), 3.90 (s, 12H), 2.17 - 2.02 (m, 4H), 1.66 - 1.53 (m, 4H), 1.50 - 1.37 (m, 2H). MS m/z: 465.31 [M+H]⁺.

### Example 11. Synthesis of Compound 11

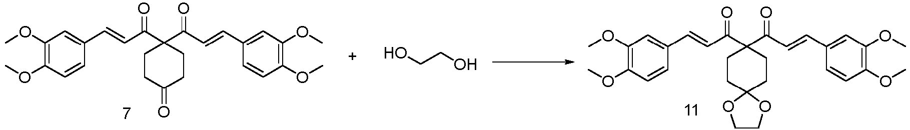

Compound 7 (500 mg, 1.04 mmol), ethylene glycol (324 mg, 5.22 mmol), acetonitrile (5 mL), and oxalic acid (94 mg, 1.04 mmol) were added to a reaction flask, and the resulting mixture was stirred at 25 °C for 20 h. After completion of the reaction, the reaction liquid was diluted with ethyl acetate (50 mL), washed with a saturated aqueous sodium bicarbonate solution, dried over sodium sulfate, concentrated, and subjected to column chromatography (petroleum ether/ethyl acetate 10:1-2:1) to give a light yellow solid (420 mg).

¹H NMR (400 MHz, CDCl₃) δ 7.66 (d, J = 15.5 Hz, 2H), 7.12 (dd, J = 8.3, 2.0 Hz, 2H), 7.00 (d, J = 1.9 Hz, 2H), 6.82 (d, J = 8.4 Hz, 2H), 6.75 (d, J = 15.4 Hz, 2H), 3.93 (s, 4H), 3.89 (s, 12H), 2.36 - 2.22 (m, 4H), 1.82 - 1.68 (m, 4H). MS m/z: 523.33 [M+H]⁺.

### Example 12. Synthesis of Compound 12 and Hydrochloride Thereof

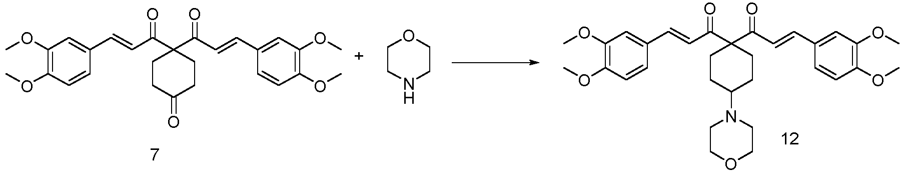

Compound 7 (200 mg, 0.4 mmol), acetic acid (25 mg, 0.4 mmol), 1,2-dichloroethane (1.5 mL), and morpholine (36 g, 0.4 mmol) were added to a reaction flask. Sodium triacetoxyborohydride (106 mg, 0.5 mmol) was added in one portion into the mixture, and the resulting mixture was stirred at 25 °C for 7 h. The reaction liquid was washed with a saturated aqueous sodium bicarbonate solution, dried over sodium sulfate, concentrated, and subjected to preparative TLC (petroleum ether/ethyl acetate 1:1) to give a light yellow solid (30 mg).

¹H NMR (400 MHz, CDCl₃) 7.68 (d, J = 15.5 Hz, 1H), 7.66 (d, J = 15.5 Hz, 1H), 7.16 - 7.09 (m, 2H), 7.03 - 6.98 (m, 2H), 6.83 (dd, J = 8.4, 3.1 Hz, 2H), 6.75 (d, J = 15.5 Hz, 1H), 6.69 (d, J = 15.5 Hz, 1H), 3.90 (s, 12H), 3.88 - 3.72 (m, 4H), 2.87 - 2.40 (m, 7H), 2.10 - 1.45 (m, 6H). MS m/z: 550.48 [M+H]⁺.

### Synthesis of compound 12 hydrochloride:

Compound 12 (200 mg), methanol (0.5 mL) and ethyl acetate (5 mL) were added to a reaction flask. The resulting mixture was stirred and dissolved. Hydrochloric acid-ethyl acetate (1 mol/L, 0.5 mL) was added into the mixture. The mixture was filtered, and the filtrate was dried under vacuum to give a light yellow solid (120 mg).

¹H NMR (400 MHz, CDCl₃) δ 12.96 (brs, 1H), 7.72 (d, J = 15.2 Hz, 1H), 7.69 (d, J = 15.3 Hz, 1H), 7.17 - 7.10 (m, 2H), 7.03 - 6.96 (m, 2H), 6.84 (dd, J = 8.4, 3.5 Hz, 2H), 6.71 (d, J = 15.4 Hz, 1H), 6.62 (d, J = 15.4 Hz, 1H), 4.56 - 4.29 (m, 2H), 4.05 - 3.80 (m, 2H), 3.91 (s, 12H), 3.34 - 2.88 (m, 5H), 2.83 - 2.68 (m, 2H), 2.43 - 2.27 (m, 2H), 1.91 - 1.69 (m, 4H). MS m/z: 550.48 [M+H]⁺.

### Example 13. Synthesis of Compound 13

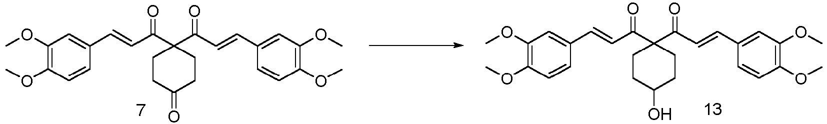

Compound 7 (200 mg, 0.4 mmol), acetic acid (25 mg, 0.4 mmol) and 1,2-dichloroethane (5 mL) were added to a reaction flask. Sodium triacetoxyborohydride (106 mg, 0.5 mmol) was added in one portion into the mixture, and the resulting mixture was stirred at 25 °C for 5 h. The reaction liquid was washed with a saturated aqueous sodium bicarbonate solution, dried over sodium sulfate, concentrated, and subjected to preparative TLC (petroleum ether/ethyl acetate 2:1) to give a light yellow solid (20 mg).

¹H NMR (400 MHz, CDCl₃) *δ* 7.68 (d, J = 15.5 Hz, 1H), 7.66 (d, J = 15.5 Hz, 1H), 7.17 - 7.10 (m, 2H), 7.03 - 6.99 (m, 2H), 6.83 (dd, J = 8.3, 2.6 Hz, 2H), 6.76 (d, J = 15.4 Hz, 1H), 6.73 (d, J = 15.5 Hz, 1H), 3.90 (s, 12H), 3.79 - 3.69 (m, 1H), 2.00 - 1.86 (m, 4H), 1.72 - 1.50 (m, 4H). MS m/z: 481.24 [M+H]⁺.

### Example 14. Synthesis of Compound 14 and Hydrochloride Thereof

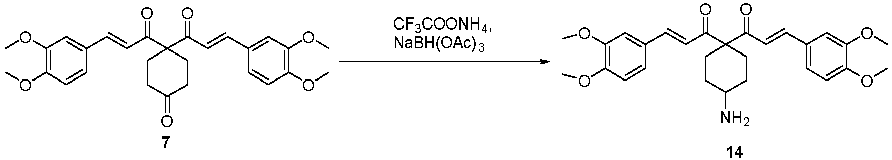

Under nitrogen atmosphere, compound 7 (300 mg, 0.63 mmol), ammonium trifluoroacetate (160 mg, 1.25 mmol), and tetrahydrofuran (10 mL) were added to a reaction flask. The resulting mixture was stirred at 25 °C for 30 mins, and sodium triacetoxyborohydride (270 mg, 1.25 mmol) was then added. The mixture was reacted at room temperature for 5 h, and ethyl acetate (30 mL) and diluted hydrochloric acid (0.2 N/30 mL) were added. Liquid separation was performed. The organic phase was washed with purified water, dried over sodium sulfate, and concentrated to give a yellow oil, which was subjected to preparative HPLC (acetonitrile/water 10:90-80:20) to give compound 14 (MS m/z: 480.26. [M+H]⁺) as a yellow solid.

Compound 14, ethyl acetate (7 mL) and methanol (0.1 mL) were added to a reaction flask. The resulting mixture was stirred and dissolved at room temperature, and hydrochloric acid-ethyl acetate (0.5 N/1.2 mL) was added dropwise into the mixture. The resulting mixture was filtered, and the filtrate was dried under vacuum at 40 °C to give compound 14 hydrochloride (20 mg) as a yellow solid.

¹H NMR (400 MHz, CDCl₃) *δ* 8.39 (brs, 3H), 7.68 (d, J = 15.4 Hz, 1H), 7.66 (d, J = 15.4 Hz, 1H), 7.18 - 7.06 (m, 2H), 7.05 - 6.95 (m, 2H), 6.83 (d, J = 8.4 Hz, 1H), 6.79 (d, J = 8.4 Hz, 1H), 6.73 (d, J = 15.5 Hz, 1H), 6.68 (d, J = 15.5 Hz, 1H), 3.89 (s, 6H), 3.88 (s, 3H), 3.87 (s, 3H), 3.32 - 3.10 (m, 1H), 2.77 - 2.52 (m, 2H), 2.31 - 2.06 (m, 2H), 1.99 - 1.53 (m, 4H). MS m/z: 480.26. [M+H]⁺.

### Example 15. Synthesis of Compound 16 and Hydrochloride Thereof

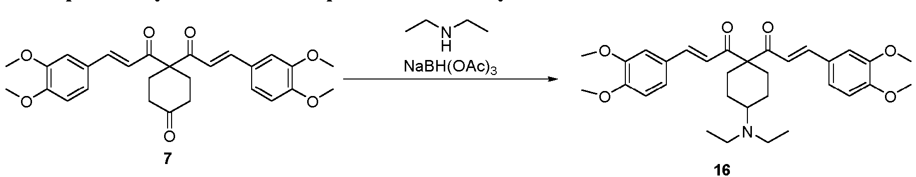

Under nitrogen atmosphere, compound 7 (300 mg, 0.63 mmol), diethylamine (9 mg, 1.25 mmol) and tetrahydrofuran (10 mL) were added to a reaction flask. The resulting mixture was stirred at 25 °C for 30 mins, and sodium triacetoxyborohydride (270 mg, 1.25 mmol) was then added. The mixture was reacted at room temperature for 5 h, and ethyl acetate (30 mL) and diluted hydrochloric acid (0.2 N/30 mL) were added. Liquid separation was performed. The organic phase was washed with purified water, dried over sodium sulfate, and concentrated to give a yellow oily compound, which was subjected to preparative TLC (petroleum ether/ethyl acetate 1:1) to give compound 16 (MS m/z: 536.44 [M+H]⁺) as a light yellow solid.

Compound 16, ethyl acetate (12 mL) and methanol (0.2 mL) were added to a reaction flask. The resulting mixture was stirred and dissolved at room temperature, and hydrochloric acid-ethyl acetate (0.5 N/1.2 mL) was added dropwise into the mixture. The resulting mixture was filtered, and the filtrate was dried under vacuum at 40 °C to give a yellow solid (160 mg), which was hydrochloride of compound 16.

¹H NMR (400 MHz, CDCl₃) δ 11.95 (brs, 1H), 7.72 (d, J = 15.1 Hz, 1H), 7.69 (d, J = 14.8 Hz, 1H), 7.14 (dd, J = 8.3, 1.9 Hz, 2H), 7.04 - 6.96 (m, 2H), 6.85 (dd, J = 8.4, 2.7 Hz, 2H), 6.71 (d, J = 15.4 Hz, 1H), 6.63 (d, J = 15.4 Hz, 1H), 3.91 (s, 12H), 3.42 - 3.22 (m, 1H), 3.18 - 2.96 (m, 4H), 2.82 - 2.66 (m, 2H), 2.37 - 2.22 (m, 2H), 1.92 - 1.67 (m, 4H), 1.51 (t, J = 7.2 Hz, 6H). MS m/z: 536.44 [M+H]⁺.

### Example 16. Synthesis of Compound 18

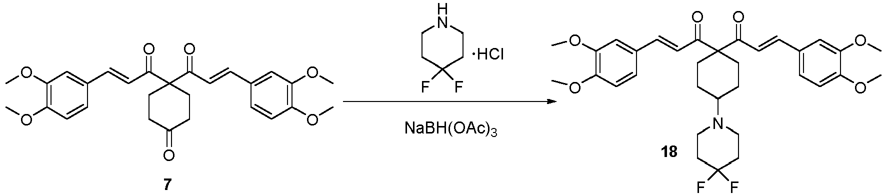

Under nitrogen atmosphere, compound 7 (300 mg, 0.63 mmol), 3,3-difluorocyclobutanamine hydrochloride (200 mg, 1.25 mmol), triethylamine (130 mg, 1.25 mmol), and tetrahydrofuran (10 mL) were added to a reaction flask. The resulting mixture was stirred at 25 °C for 30 min, and sodium triacetoxyborohydride (270 mg, 1.25 mmol) was then added. The mixture was reacted at room temperature for 2 h, and ethyl acetate (30 mL) and diluted hydrochloric acid (0.2 N/30 mL) were added. Liquid separation was performed. The organic phase was washed with purified water, dried over anhydrous sodium sulfate, concentrated, and subjected to preparative TLC (petroleum ether/ethyl acetate 1:1) to give compound 18 (6 mg) as a light yellow solid. MS m/z: 584.50 [M+H]⁺. ¹H NMR (400 MHz, CDCl₃) δ 7.68 (d, J = 15.5 Hz, 1H), 7.65 (d, J = 15.5 Hz, 1H), 7.17 - 7.06 (m, 2H), 7.01 (d, J = 2.0 Hz, 1H), 7.00 (d, J = 2.0 Hz, 1H), 6.83 (dd, J = 8.4, 3.4 Hz, 2H), 6.76 (d, J = 15.5 Hz, 1H), 6.70 (d, J = 15.5 Hz, 1H), 3.90 (s, 12H), 2.72 - 2.56 (m, 2H), 2.50 - 2.36 (m, 1H),2.06 - 1.88(m,4H), 1.80 - 1.41 (m, 10H).

¹⁹F NMR (376 MHz, CDCl₃) δ -97.80.

### Example 17. Synthesis of Compound 19 and Hydrochloride Thereof

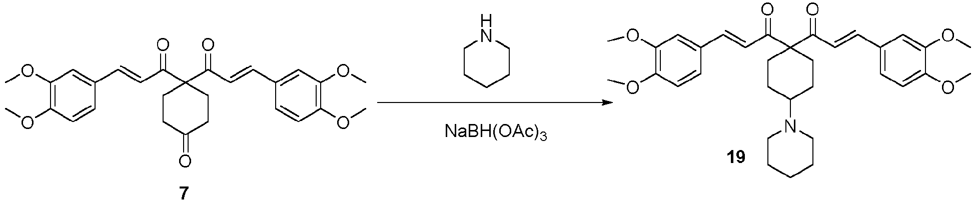

Under nitrogen atmosphere, compound 7 (300 mg, 0.63 mmol), piperidine (110 mg, 1.25 mmol), and tetrahydrofuran (10 mL) were added to a reaction flask. The resulting mixture was stirred at 25 °C for 30 mins, and sodium triacetoxyborohydride (270 mg, 1.25 mmol) was then added. The mixture was reacted at room temperature for 3 h, and ethyl acetate (30 mL) and diluted hydrochloric acid (0.2 N/30 mL) were added. Liquid separation was performed. The organic phase was washed with purified water, dried over sodium sulfate, and concentrated to give a yellow oily compound, which was subjected to preparative TLC (petroleum ether/ethyl acetate 1:1) to give compound 19 (MS m/z: 548.45 [M+H]⁺) as a light yellow solid.

Compound 19, ethyl acetate (12 mL) and methanol (0.2 mL) were added to a reaction flask. The resulting mixture was stirred and dissolved at room temperature, and hydrochloric acid-ethyl acetate (0.5 N/1.2 mL) was added dropwise. The resulting mixture was filtered, and the filtrate was dried under vacuum at 40 °C to give compound 19 hydrochloride (120 mg) as a yellow solid.

¹H NMR (400 MHz, CDCl₃) δ 11.95 (brs, 1H), 7.71 (d, J = 15.4 Hz, 1H), 7.68 (d, J = 15.4 Hz, 1H), 7.14 (dd, J = 8.4, 2.0 Hz, 2H), 7.05 - 6.96 (m, 2H), 6.84 (dd, J = 8.4, 3.3 Hz, 2H), 6.71 (d, J = 15.4 Hz, 1H), 6.63 (d, J = 15.4 Hz, 1H), 3.91 (s, 12H), 3.45 - 3.28 (m, 2H), 3.17 - 3.03 (m, 1H), 2.86 - 2.62 (m, 4H), 2.56 - 2.29 (m, 4H), 2.00 - 1.52 (m, 8H).MS m/z: 548.45 [M+H]⁺.

### Example 18. Synthesis of Compound 22

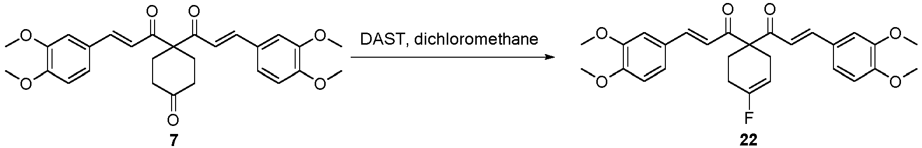

Under nitrogen atmosphere, compound 7 (1.00 g, 2.09 mmol) and dichloromethane (20 mL) were added to a reaction flask. The resulting mixture was stirred at 0 °C for 10 mins, and diethylaminosulfur trifluoride (0.40 g, 2.51 mmol) was added dropwise. The mixture was then reacted at room temperature overnight, and a saturated aqueous sodium bicarbonate solution was added. Liquid separation was performed. The aqueous phase was extracted with dichloromethane, concentrated, and then subjected to column chromatography (petroleum ether/ethyl acetate 10:1-2:1) to give a crude product (320 mg), which was subjected to preparative HPLC (acetonitrile/water 10:90-80:20) to give a light yellow solid (0.10 g).

¹H NMR (400 MHz, CDCl₃) δ 7.704 (d, J = 15.4 Hz, 2H), 7.138 (dd, J = 8.3, 1.9 Hz, 2H), 7.013 (d, J = 1.9 Hz, 2H), 6.842 (d, J = 8.3 Hz, 2H), 6.727 (d, J = 15.4 Hz, 2H), 5.326 - 5.169 (m, 1H), 3.910 (s, 6H), 3.904 (s, 6H), 2.822 - 2.606 (m, 2H), 2.469 - 2.352 (m, 2H), 2.317 - 2.198 (m, 2H). ¹⁹F NMR (377 MHz, CDCl₃) δ -102.82. MS m/z: 481.99 [M+H]⁺.

### Example 19. Synthesis of Compound 23 and Hydrochloride Thereof

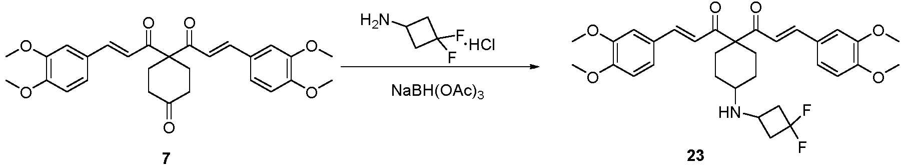

Under nitrogen atmosphere, compound 7 (300 mg, 0.63 mmol), 3,3-difluorocyclobutanamine hydrochloride (180 mg, 1.25 mmol), triethylamine (130 mg, 1.25 mmol), and tetrahydrofuran (10 mL) were added to a 50 mL reaction flask. The resulting mixture was stirred at 25 °C for 30 mins, and sodium triacetoxyborohydride (270 mg, 1.25 mmol) was then added. The mixture was reacted at room temperature for 2 h. Ethyl acetate (30 mL) and diluted hydrochloric acid (0.2 N/30 mL) were added. Liquid separation was performed. The organic phase was washed with purified water, dried over anhydrous sodium sulfate, and concentrated to give compound 23 (MS m/z: 570.43 [M+H]⁺) as a yellow oil.

Compound 23, ethyl acetate (15 mL) and methanol (0.3 mL) were added. The mixture was stirred and dissolved at room temperature, and hydrochloric acid-ethyl acetate (0.5 N/1.2 mL) was added dropwise. The resulting mixture was filtered, and the filtrate was dried under vacuum at 40 °C to give compound 23 hydrochloride (120 mg) as a yellow solid.

¹H NMR (400 MHz, CDCl₃) δ 10.26 (brs, 2H), 7.71 (d, J = 15.4 Hz, 1H), 7.67 (d, J = 15.4 Hz, 1H), 7.18 - 7.06 (m, 2H), 7.03 - 6.97 (m, 2H), 6.82 (d, J = 8.4 Hz, 1H), 6.79 (d, J = 8.4 Hz, 1H), 6.74 (d, J = 15.4 Hz, 1H), 6.69 (d, J = 15.5 Hz, 1H), 3.88 (s, 12H), 3.66 - 3.50 (m, 1H), 3.41 - 3.22 (m, 2H), 3.13 - 2.88 (m, 3H), 2.81 - 2.60 (m, 2H), 2.28 - 2.13 (m, 2H), 1.94 - 1.73 (m, 4H).MS m/z: 570.43 [M+H]⁺.

### Example 20. Synthesis of Compound 24

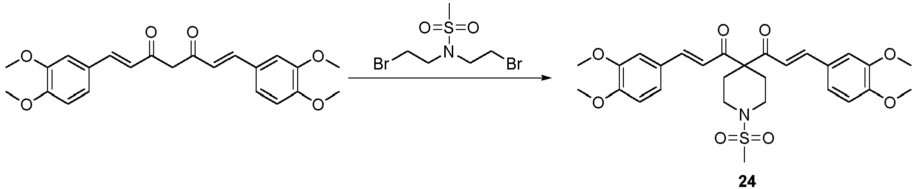

Dimethylcurcumin (500 mg, 1.26 mmol), dibromoethyl methanesulfonamide (460 mg, 1.50 mmol), K₂CO₃ (520 mg, 3.76 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phase was washed with saturated brine to remove DMF, dried over anhydrous sodium sulfate, concentrated, and subjected to column chromatography (petroleum ether/ethyl acetate 10:1-2:1) to give a light yellow solid (20 mg). MS m/z: 544.42 [M+H]⁺.

¹H NMR (400 MHz, CDCl₃) δ 7.72 (d, J = 15.4 Hz, 2H), 7.15 (dd, J = 8.4, 2.0 Hz, 2H), 7.01 (d, J = 2.0 Hz, 2H), 6.85 (d, J = 8.3 Hz, 2H), 6.69 (d, J = 15.4 Hz, 2H), 3.91 (s, 12H), 3.36 - 3.27 (m, 4H), 2.76 (s, 3H), 2.42 - 2.28 (m, 4H).

### Example 21. Synthesis of Compound 25

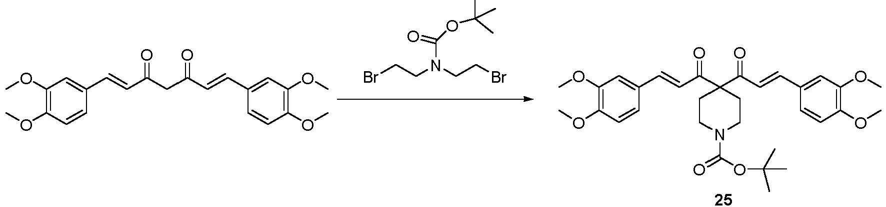

Dimethylcurcumin (500 mg, 1.26 mmol), tert-butyl bis(2-bromoethyl)carbamate (500 mg, 1.50 mmol), K₂CO₃ (520 mg, 3.76 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phase was washed with saturated brine to remove DMF, dried over anhydrous sodium sulfate, concentrated, and subjected to column chromatography (petroleum ether/ethyl acetate 10:1-2:1) to give a light yellow solid (90 mg).

MS m/z: 566.47 [M+H]⁺.

¹H NMR (400 MHz, CDCl₃) δ 7.69 (d, J = 15.4 Hz, 2H), 7.13 (dd, J = 8.4, 2.0 Hz, 2H), 7.00 (d, J = 2.0 Hz, 2H), 6.83 (d, J = 8.4 Hz, 2H), 6.70 (d, J = 15.5 Hz, 2H), 3.90 (s, 12H), 3.54 - 3.26 (m, 4H), 2.30 - 2.10 (m, 4H), 1.44 (s, 9H).

### Example 22. Synthesis of Compound 26

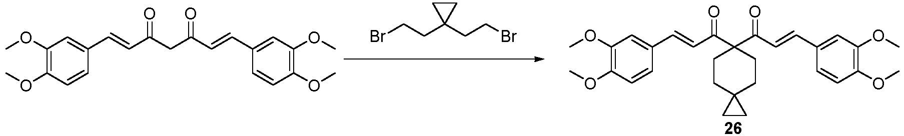

Dimethylcurcumin (500 mg, 1.26 mmol), (dibromoethyl)cyclopropane (390 mg, 1.52 mmol), K₂CO₃ (520 mg, 3.76 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phase was washed with saturated brine to remove DMF, dried over anhydrous sodium sulfate, concentrated, and subjected to column chromatography (petroleum ether/ethyl acetate 10:1-2:1) to give a light yellow solid (50 mg).

¹H NMR (400 MHz, CDCl₃) δ 7.69 (s, 2H), 7.13 (dd, *J =* 8.3, 1.9 Hz, 2H), 7.01 (d, *J =* 2.0 Hz, 2H), 6.83 (d, *J =* 8.3 Hz, 2H), 6.77 (d, *J =* 15.5 Hz, 2H), 3.90 (s, 12H), 2.26 - 2.13 (m, 4H), 1.45 - 1.37 (m, 4H), 0.25 (s, 4H). MS m/z: 491.42 [M+H]⁺.

### Example 23. Synthesis of Compound 27

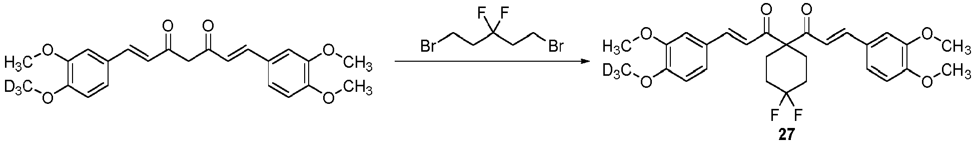

Starting material B (500 mg, 1.25 mmol), 1,5-dibromo-3,3-difluoropentane (317 mg, 1.2 mmol), K₂CO₃ (520 mg, 3.76 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phase was washed with saturated brine to remove DMF, dried over anhydrous sodium sulfate, concentrated, and subjected to column chromatography (petroleum ether/ethyl acetate 10:1-2:1) to give a light yellow solid (30 mg).

¹H NMR (400 MHz, CDCl₃) δ 7.707 (d, J = 15.5 Hz, 2H), 7.139 (dd, J = 8.3, 2.0 Hz, 2H), 7.009 (d, J = 2.0 Hz, 2H), 6.840 (dd, J = 8.4, 2.4 Hz, 2H), 6.722 (d, J = 15.4 Hz, 2H), 3.907 (s, 6H), 3.903(s, 3H), 2.38 - 2.27 (m, 4H), 2.12 - 1.85 (m, 4H). MS m/z: 504.44 [M+H]⁺.

### Example 24. Synthesis of Compound 28

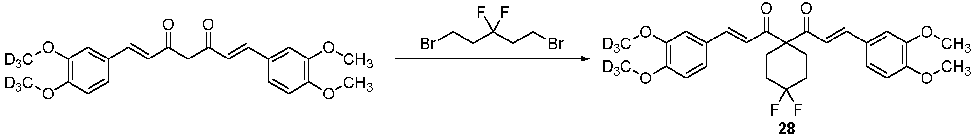

Starting material C (500 mg, 1.24 mmol), 1,5-dibromo-3,3-difluoropentane (317 mg, 1.2 mmol), K₂CO₃ (520 mg, 3.76 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phase was washed with saturated brine to remove DMF, dried over anhydrous sodium sulfate, concentrated, and subjected to column chromatography (petroleum ether/ethyl acetate 10:1-2:1) to give a light yellow solid (35 mg).

¹H NMR (400 MHz, CDCl₃) δ 7.709 (d, J = 15.4 Hz, 2H), 7.140 (dd, J = 8.3, 2.0 Hz, 2H), 7.027 - 6.982 (m, 2H), 6.843 (dd, J = 8.4, 2.5 Hz, 2H), 6.722 (d, J = 15.4 Hz, 2H), 3.910 (s, 3H), 3.907 (s, 3H), 2.372 - 2.227 (m, 4H), 2.116 -1.949 (m, 4H). MS m/z: 507.46 [M+H]⁺.

### Example 25. Synthesis of Compound 29

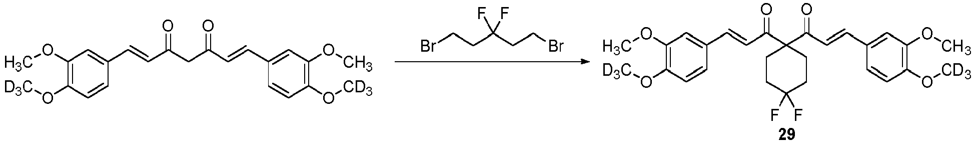

Starting material D (500 mg, 1.24 mmol), 1,5-dibromo-3,3-difluoropentane (317 mg, 1.2 mmol), K₂CO₃ (520 mg, 3.76 mmol), and DMF (15 mL) were added to a reaction flask, and the resulting mixture was stirred at 50 °C for 20 h. After completion of the reaction, water (50 mL) and ethyl acetate (20 mL) were added. Liquid separation was performed. The aqueous phase was extracted with ethyl acetate (20 mL). The organic phase was washed with saturated brine to remove DMF, dried over anhydrous sodium sulfate, concentrated, and subjected to column chromatography (petroleum ether/ethyl acetate 10:1-2:1) to give a light yellow solid (25 mg).

¹H NMR (400 MHz, CDCl₃) δ 7.704 (d, J = 15.4 Hz, 2H), 7.136 (dd, J = 8.4, 2.0 Hz, 2H), 7.007 (d, J = 2.0 Hz, 2H), 6.834 (d, J = 8.3 Hz, 2H), 6.720 (d, J = 15.4 Hz, 2H), 3.904 (s, 6H), 2.366 - 2.227 (m, 4H), 2.117 - 1.939 (m, 4H). MS m/z: 507.51 [M+H]⁺.

### Example 26. Synthesis of Compound 30 and Hydrochloride Thereof

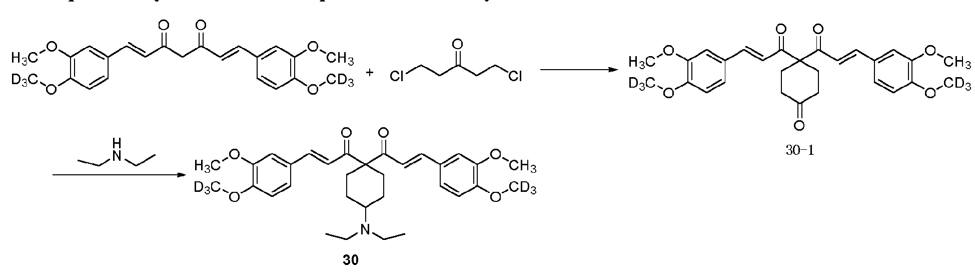

### Step 1: synthesis of intermediate 30-1

Starting material D (1.0 g, 2.50 mmol, 1.0 eq), 1,5-dichloropentanone (0.391 g, 2.52 mmol), KBr (1.20 g, 10.1 mmol), K₂CO₃ (1.05 g, 7.57 mmol), and DMF (30 mL) were added to a reaction flask, and the resulting mixture was stirred at 50 °C for 20 h. After completion of the reaction, water (70 mL) and ethyl acetate (40 mL) were added. Liquid separation was performed. The aqueous phase was extracted with ethyl acetate (400 mL). The organic phase was washed with saturated brine to remove DMF, dried over anhydrous sodium sulfate, concentrated, and subjected to column chromatography (petroleum ether/ethyl acetate 10:1-2:1) to give a yellow solid (600 mg).

### Step 2: synthesis of compound 30 and hydrochloride thereof

Under nitrogen atmosphere, intermediate 30-1 (300 mg, 0.62 mmol), diethylamine (9 mg, 1.25 mmol), and tetrahydrofuran (10 mL) were added to a reaction flask. The resulting mixture was stirred at 25 °C for 30 mins, and sodium triacetoxyborohydride (270 mg, 1.25 mmol) was then added. The mixture was reacted at room temperature for 5 h. Ethyl acetate (30 mL) and diluted hydrochloric acid (0.2 N/30 mL) were added. Liquid separation was performed. The organic phase was washed with purified water, dried over sodium sulfate, and concentrated to give a yellow oily compound, which was subjected to preparative TLC (petroleum ether/ethyl acetate 1:1) to give compound 30 (MS m/z: 542.57 [M+H]⁺) as a light yellow solid.

Compound 30, ethyl acetate (12 mL) and methanol (0.2 mL) were added to a reaction flask. The resulting mixture was stirred and dissolved at room temperature, and hydrochloric acid-ethyl acetate (0.5 N/1.2 mL) was added dropwise. The resulting mixture was filtered, and the filtrate was dried under vacuum at 40 °C to give hydrochloride of compound 30 (60 mg) as a yellow solid.

¹H NMR (400 MHz, CDCl₃) δ 11.83 (brs, 1H), 7.77 - 7.60 (m, 2H), 7.14 (d, J = 8.2 Hz, 2H), 7.01 (dd, J = 4.2, 1.8 Hz, 2H), 6.84 (dd, J = 8.3, 2.7 Hz, 2H), 6.72 (d, J = 15.4 Hz, 1H), 6.63 (d, J = 15.4 Hz, 1H), 3.91 (s, 6H), 3.37 - 3.20 (m, 1H), 3.17 - 2.88 (m, 4H), 2.82 - 2.63 (m, 2H), 2.37 - 2.19 (m, 2H), 1.91 - 1.63 (m, 4H), 1.56 - 1.37 (m, 6H).MS m/z: 542.57 [M+H]⁺.

### Test Example 1. Cell Viability Inhibition Assay of Compounds of the Present Disclosure on Human Prostate Cancer Cells

Human prostate cancer cells LNCaP and human prostate cancer cells 22Rvl are present in patients with prostate cancer. An androgen, DHT, is able to promote the growth of human prostate cancer cells LNCaP. Using the cell model described above was intended to study the inhibitory effects of the compounds of the present disclosure on the growth ofhuman prostate cancer cells (human prostate cancer cells LNCaP and human prostate cancer cells 22Rvl) in the presence or absence of DHT.

**Assay materials:** Test compounds (prepared by the method of the present disclosure), human prostate cancer cells LNCaP (American Type Culture Collection (ATCC), Cat No: CRL-1740), human prostate cancer cells 22Rvl (American Type Culture Collection (ATCC), Cat No: CRL-2505), RPMI 1640 medium (Invitrogen; Cat. No. 11875119), fetal bovine serum (Certified FBS Charcoal Stripped, Biolohivsl Industries, Cat. No. 04-204-1A), penicillin-streptomycin mixed solution (Solarbio; Cat No: P1400), and CellTiter-Glo (CTG) reagent (Promega, Cat# G7573).

**Assay method:** Human prostate cancer cells LNCaP and human prostate cancer cells 22Rvl in the exponential growth phase were placed under a microscope, and the growth state of the cells was observed to be good. The medium in the culture dish was discarded, and 5 mL of trypsin was added for digestion for about 3 mins. The digestion was stopped by adding 10 mL of a fresh medium. The cells were fully pipetted and transferred to a 15 mL centrifuge tube, and the cells were collected by centrifugation at 1000 rpm for 5 mins. The cells collected by centrifugation were resuspended in 11 mL of a fresh medium. 1 mL of the cells was taken and counted, and the cell viability was detected. The cell density was adjusted to 1 × 10⁴ cells/mL by adding an appropriate amount of the medium. The cells were seeded in a 96-well plate at 200 µL/well, and the plate was incubated overnight in a cell incubator.

After the cells were attached to the well, for the human prostate cancer cells LNCaP group, test compounds (0.1 µM, 0.3 µM, 1 µM, 3 µM, 10 µM, and 30 µM) and DHT (1 nM, inducing the expression of AR) were added to the 96-well plate, and the plate was incubated for another 5 days; for the human prostate cancer cells 22Rvl group, test compounds (0.1 µM, 0.3 µM, 1 µM, 3 µM, 10 µM, and 30 µM) were added to the 96-well plate, and the plate was incubated for another 5 days. After 5 days, the 96-well plate was taken out from the incubator and observed under the microscope for no abnormality in the cell state. The 96-well plate was equilibrated at room temperature for 30 mins.

The cell viability was detected by using a CTG method. Before detection, CellTiter-Glo^{®} Buffer and a substrate thereof (collectively called CTG reagent) were thawed and equilibrated to room temperature. Then, 100 mL of Buffer was gently mixed with the substrate to form a homogeneous solution. The homogeneous solution was added to the 96-well plate at 100 µL/well, and the 96-well plate was incubated on a shaker for 15 mins. The fluorescence value in each well was then detected. The detection method for the fluorescence value in each well was as follows: after adding the CTG reagent, luminescence detection (integration time: 500 ms) was performed by using a correspondingly equipped multi-mode microplate reader (M200Pro, TECAN, Switzerland) according to the supplier's manual to quantify the effect of the inhibitor on the cell viability. For data analysis, an assay background value was subtracted from all data points, which was determined in wells containing the medium but no cells. Data were processed using the XLfit software (XLfit 5.2., IDBS, UK), and IC₅₀ values of the test compounds were calculated. The IC₅₀ values of the typical compounds of the present disclosure on human prostate cancer cells LNCaP and human prostate cancer cells 22Rvl are shown in Table 1 below.

**Table 1. Inhibitory activities of compounds of the present disclosure against human prostate cancer cells LNCaP and human prostate cancer cells 22Rvl**

| **Compound** | **Inhibitory activity against human prostate cancer cell LNCaP** IC₅₀ (µM) | **Inhibitory activity against human prostate cancer cells 22Rvl** IC₅₀ (µM) |
|---|---|---|
| 1 | 1.71 | - |
| 2 | 1.82 | - |
| 3 | 1.75 | - |
| 4 | 1.77 | - |
| 5 | 5.48 | - |
| 6 | 2.56 | - |
| 7 | 0.83 | - |
| 8 | 0.61 | 0.35 |
| 9 | 3.31 | - |
| 10 | 1.71 | - |
| 11 | 0.98 | - |
| 12 | 1.29 | - |
| 12 hydrochloride | 0.45 | |
| 13 | 0.95 | - |
| 16 hydrochloride | 0.37 | - |
| 18 hydrochloride | 0.61 | - |
| 19 hydrochloride | 0.35 | - |
| 22 | 3.14 | - |
| 23 hydrochloride | 0.62 | - |
| 24 | 0.61 | - |
| 25 | 1.37 | - |
| 26 | 0.62 | - |

| | | |
|---|---|---|
| "-" indicates that the relevant assay was not performed. | | |

As can be seen from the assay data in the table above, the compounds of the present disclosure had good inhibitory activities against the growth of prostate cancer cells, and were able to effectively inhibit the growth of prostate cancer cells.

### Test Example 2. Test of Effects of Compounds of the Present Disclosure on AR Protein Expression in Human Prostate Cancer Cells LNCaP

AR is a key factor for regulating the response of prostate cancer cells to androgens. AR is also a key factor for the response of hair follicles or tissues around the hair follicles to androgens, and the reduction of the AR content not only can down-regulate the growth of the prostate cancer cells, but also can inhibit androgenetic alopecia. This test example is to test the effects of the compounds of the present disclosure on the reduction of AR protein expression in human prostate cancer cells LNCaP in the presence of DHT. The compounds of the present disclosure were analyzed for anti-AR activity in human prostate cancer cells LNCaP by measuring the reduction of AR protein using the Western blot method.

**Assay materials:** Test compounds (prepared by the method of the present disclosure), human prostate cancer cells LNCaP (American Type Culture Collection (ATCC), Cat No: CRL-1740), RPMI 1640 medium (Invitrogen; Cat. No. 11875119), fetal bovine serum (Certified FBS Charcoal Stripped, Biolohivsl Industries, Cat. No. 04-204-1A), BCA protein quantification kit (Thermo; Cat. No. 23225), color prestained protein marker (Beyotime; Cat. No. P0069), GAPDH antibody (Millipore; Cat. No. MAB374), and AR antibody (CST; Cat. No. 5153).

**Assay method:** Human prostate cancer cells LNCaP were seeded in a 6-well plate at 4 × 10⁵ cells/mL and at a volume of 2 mL per well. The plate was incubated overnight in a cell incubator. DMSO and dihydrotestosterone (DHT, 1 nM) were used as control groups. Assays were performed in the presence of dihydrotestosterone (DHT, 1 nM). After incubating with the test compounds for a specified time, the cells were collected and dissolved according to the Western blot technique known in the biochemical art. A DMSO group, a DHT group (1 nM) and test compound groups (containing 1 nM DHT) were set during the assay. DMSO and DHT (1 nM DHT) were added to 6-well plates corresponding to the DMSO group and the DHT group, respectively. DHT (1 nM) and the test compounds (0.6 µM, 1.2 µM, 2.5 µM, 5 µM, and 10 µM) were added to 6-well plates corresponding to the test compound groups. After cells in the control groups and the test compound groups were incubated for 48 h, the cells were collected for lysis. The protein concentration was quantified using the BCA kit, and then the samples were stored for later use.

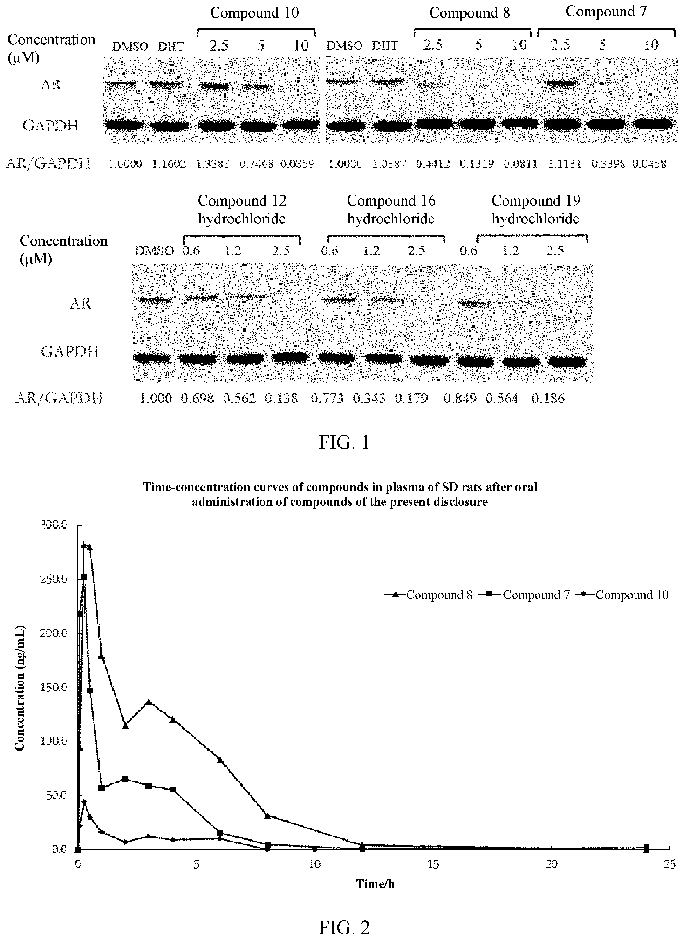

The content of the AR protein in each group was determined by using Western Blot, and the loading amount of the sample in each group was 30 µg. Western blot analysis has been disclosed in the prior art. Specifically, the cells were collected in 2× sodium dodecyl sulfate/polyacrylamide gel electrophoresis loading buffer or in radioimmunoprecipitation assay (RIPA) buffer strengthened with 10 µg/mL benzamidine, 10 µg/mL trypsin inhibitor, and 1 mM phenylmethylsulfonyl fluoride. A sample of total protein (about 40 µg) from each cell lysate was separated by electrophoresis on an SDS/PAGE gel. After separation by electrophoresis, the proteins were transferred from the gel to a nitrocellulose membrane following the standard procedures. The membrane was then incubated with 10% non-fat milk in phosphate-buffered saline supplemented with 0.1% Tween 20 (PBST) for 1 h, followed by incubation with a primary human AR-specific antibody (purchased from BD-Harlingen) at 4 °C overnight. After incubation, the membrane was washed 3 times with PBST buffer, each for 10 mins. An alkaline phosphatase-conjugated secondary antibody was then added, and the membrane was incubated at room temperature for 1 h. After the secondary incubation, the membrane was washed again with PBST, and the AR protein signal in the membrane was visualized by adding an alkaline phosphatase medium, bromochloroindolyl phosphate, and nitrotetrazole to the membrane. To ensure that an equal amount of protein from each sample was analyzed, a portion of the membrane was kept with a specific antibody for the regulatory protein GAPDH (Santa Cruz Biotechnology), and the GAPDH signal was visualized with the secondary antibody described above. The protein signal intensity (as indicated by color bands on the membrane) was measured using a densitometer and analyzed by the NIH Image J software (NIH1.33). The AR protein signal in each sample was normalized (relative to GAPDH), and the data were presented as the ratio of AR grey values to GAPDH grey values, as shown in FIG. 1.

The above experiments tested the ability of some compounds of the present disclosure to reduce AR expression (i.e., anti-AR activity) in human prostate cancer cells LNCaP, and the anti-AR activity of each compound was compared to the DHT and DMSO control groups at various concentrations. The relative potency of the anti-AR activity of the compounds of the present disclosure was expressed as the AR reduction percentage after the compounds were incubated at concentrations of 2.5 µM, 5 µM, and 10 µM for 48 h (with 1 nM DHT). The evaluation index is shown in Table 2 below:

**Table 2. Evaluation of relative potency**

| Relative potency | AR reduction percentage (%) |
|---|---|
| + | 12.5<*≤25 |
| ++ | 25<*≤37.5 |
| +++ | 37.5<*≤50 |
| ++++ | 50<*≤62.5 |
| +++++ | 62.5<*≤75 |
| ++++++ | 75<*≤100 |

The AR reduction percentage (%) was calculated as follows: AR reduction percentage (%) = (AR in DHT group /GAPDH value - AR in test compound group/GAPDH value)/AR in DHT group/GAPDH value × 100%.

The ability of the compounds of the present disclosure to reduce AR expression (i.e., anti-AR activity) in the human prostate cancer cells LNCaP is shown in Table 3. The values in Table 3 refer to AR reduction percentage (%), the concentrations in Table 3 refer to the concentrations of the test compounds, and a greater number of "+" in the relative potency column in Table 3 indicates better anti-AR activity of the compound.

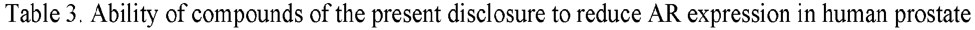

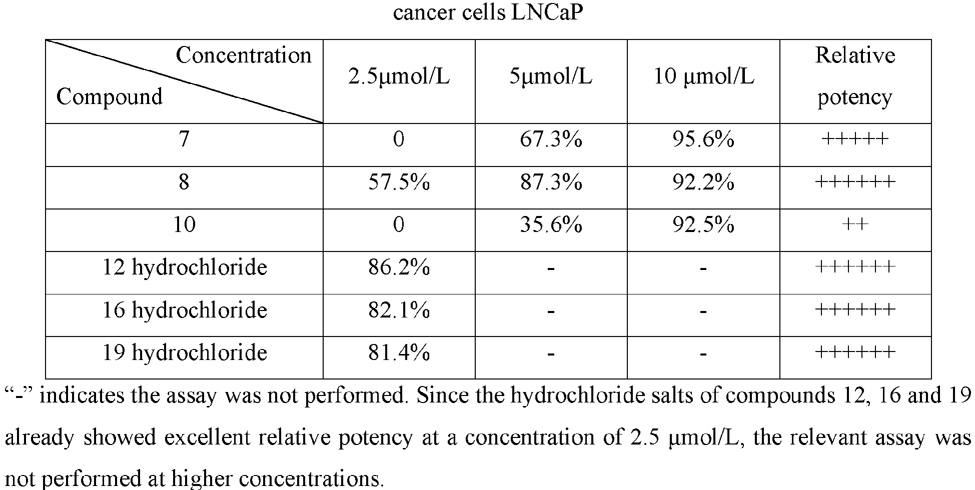

**Conclusion:** DHT could up-regulate the expression of AR protein in human prostate cancer cells LNCaP, and the compounds of the present disclosure could inhibit the expression of AR protein in human prostate cancer cells LNCaP in a dose-dependent manner, that is, the compounds of the present disclosure have anti-AR activities. In combination with the relative potency, the compounds of the present disclosure, particularly compound 7, compound 8, compound 12 hydrochloride, compound 16 hydrochloride, and compound 19 hydrochloride, have significant anti-AR activities.

### Test example 3. Pharmacokinetic Study

This test example was intended to study single oral administration of solutions of the compounds of the present disclosure to SD rats, to detect the concentration of the active ingredient in the plasma, and to evaluate their pharmacokinetic (PK) profiles in SD rats.

Experimental materials: male SD rats (weighing 180-220 g, purchased from Beijing Vital River Laboratory Animal Technology Co., Ltd., production license No. SCXK(Beijing)2016-0006), experimental compounds (prepared according to the methods of the examples of the present disclosure), and purified water (made in-house).

Experimental method: Male SD rats were randomly grouped (3 rats per group), given ad libitum access to water during the assay, fasted for 12 or more hours before administration, and given food 4 hours after administration. These groups of SD rats were intragastrically administered, by mouth, 0.5% aqueous suspensions of the test compounds (formula comprised: 0.5% test compound, 1% CMCNa, 0.5% Tween 80, and 98% purified water) at a dose of 50 mg/kg (calculated based on the test compound).

Blood samples were collected into K₂EDTA anticoagulation tubes at 0 min before administration and at 5 min, 15 min, 30 min, 1 h, 2 h, 3 h, 4 h, 6 h, 8 h, and 12 h after administration, and then were temporarily placed on ice before centrifugation.

The blood was centrifuged within 60 min of being collected to isolate plasma (centrifuged at 8000 rpm at 2-8 °C for 5 min). After the centrifugation, the plasma was transferred to a 96-well plate or centrifuge tubes, transported in a box with ice, and stored at ≤-15 °C before LC-MS/MS analysis. The drug concentration in the plasma of SD rats was measured by LC-MS/MS bioanalysis, and the plasma concentration-time data were analyzed using WinNonlinTM (Version 8.3, Certara, USA) and a non-compartmental model to evaluate the pharmacokinetic (PK) profiles of the compounds in SD rats. The data are shown in Table 4, and the pharmacokinetic curves are shown in FIG. 2.

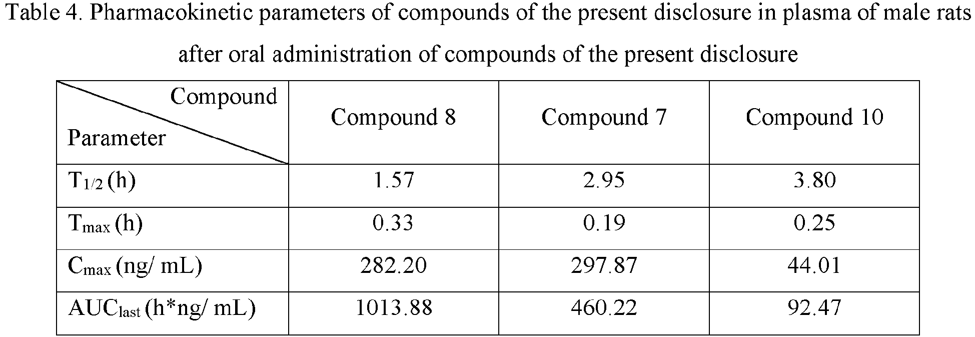

The plasma concentration-time curves in FIG. 2 show that compound 10 had the lowest Cₘₐₓ and the lowest AUCₗₐₛₜ in rats; compound 7 and compound 8 had similar Cₘₐₓ, but compound 8 had higher AUCₗₐₛₜ than that of compound 7. These results indicate that after the compounds were intragastrically administered to SD rats at the same dose, compound 10 likely exerted the worst efficacy due to the lowest exposure in the rats, followed by compound 7, and compound 8 likely exerted the best efficacy due to the significantly increased Cₘₐₓ and AUCₗₐₛₜ.

The results described above indicate that the compounds of the present disclosure had significantly improved pharmacokinetic properties. Particularly, AUC and Cₘₐₓ were both very significantly improved after administration of the compounds of the present disclosure. Therefore, the compounds of the present disclosure have good druggability and are able to obtain better efficacy at a lower administration dose.

### Test Example 4. Hair Growth Promotion Test of Compounds of the Present Disclosure in Mice with Alopecia

**Objective:** The compounds of the present disclosure were tested for their hair growth-promoting effects on an alopecia mouse model.

**Test materials:** C57BL/6 mice (purchased from SPF (Beijing) Biotechnology Co., Ltd., production license No.: SCXK (Beijing) 2019-0010), test compounds (prepared by the method of the present disclosure), rosin (Shanghai YuanYe Bio-Technology Co., Ltd., lot No. Y18M10C83144), liquid paraffin (Shanghai YuanYe Bio-Technology Co., Ltd., lot No.: Z22S11Y125555), chloral hydrate (Sinopharm Chemical Reagent Co., Ltd., lot No.: 20190823, a 2% chloral hydrate solution was prepared by using a 0.9% sodium chloride solution before use), testosterone propionate (Aladdin Reagent (Shanghai) Co., Ltd., lot No.: T101368, a 0.5% testosterone propionate solution was prepared by using an a soybean oil for injection before use), soybean oil for injection (Liaoning Shinsun Pharmaceutical Co., Ltd., lot No.: NMPA Approval No. H21024303), paraformaldehyde (Xilong Scientific Co., Ltd., lot No.: 1705022), a 0.9% sodium chloride solution (Jiangsu Huai'an Shuanghe Pharmaceutical Co., Ltd., lot No.: 210322-3C), an electronic balance (Beijing Sartorius Scientific Instruments Co., Ltd., model: BS224 S; GZX-9140MBE), and a digital display electric heating constant temperature air blast drying oven (Shanghai Boxun Medical Biological Instrument Corp.).

**Test method:** 8-week-old male C57BL/6 mice were selected and injected with 2% chloral hydrate (400 mg/kg) for anesthesia. Equal amounts of rosin and paraffin were weighed out, heated, and mixed well. The mixture was applied on the front back of the mice in an area of about 2 cm × 2.5 cm. After the mixture was cooled to be hard, the solidified hair was torn off by tweezers gently. Animals found to have a dark skin color after hair removal, indicating that they were in the anagen phase with active hair follicle growth, were excluded from the study.

Qualified C57BL/6 mice (3 mice per group) were randomly assigned to a blank group, a model group, a positive control drug group (7.5% clascoterone group), and test groups (including 0.3% compound 8 group and 0.3% compound 7 group). Mice in the blank group were coated with 0.1 mL of normal saline in the depilated area every morning (1 time a day for 17 consecutive days); mice in the model group were coated with 0.1 mL of 0.5% testosterone propionate solution in the depilated area every morning (1 time a day, starting on the first day, for 17 consecutive days), and with 0.5 mL of the control solvent in the depilated area every afternoon (1 time a day, starting on the second day, for 16 consecutive days); mice in the positive control drug group were coated with 0.1 mL of 0.5% testosterone propionate solution in the depilated area every morning (1 time a day, starting on the first day, for 17 consecutive days), and with 0.5 mL of 7.5% clascoterone solution in the depilated area every afternoon (1 time a day, starting on the second day, for 16 consecutive days); and mice in the test groups were coated with 0.1 mL of 0.5% testosterone propionate solution in the depilated area every morning (1 time a day, starting on the first day, for 17 consecutive days), and with 0.5 mL of 0.3% test compound solution in the depilated area every afternoon (1 time a day, starting on the second day, for 16 consecutive days). The formula of the test compound solution was 0.3% test compound, 40% DMSO, 30% ethanol, and the balance of water; the formula of the control solvent was 40% DMSO, 30% ethanol, and the balance of water; the formula of the positive control drug group was 7.5% clascoterone, 40% DMSO, 30% ethanol, and the balance of water. The hair regrowth in the depilated area of the mice in each group was observed and photographed 16 days after the start of topical administration of the test compound solution.

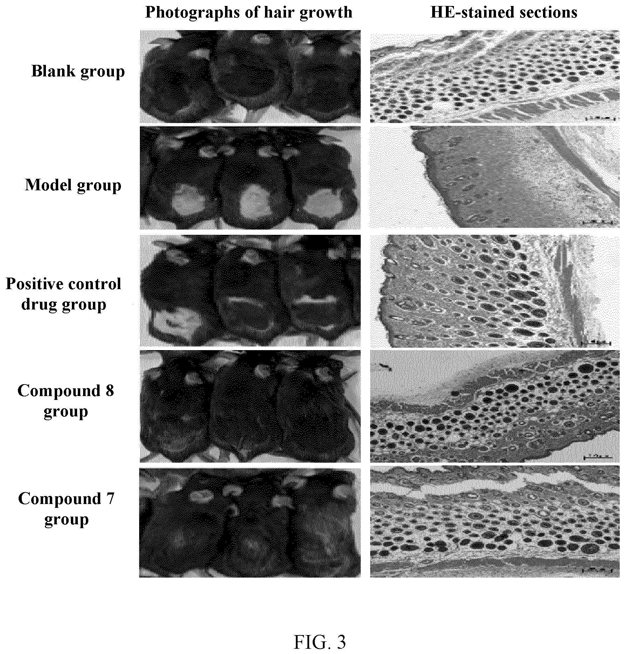

On day 18 of the test, 5 new hairs were pulled out from the back depilated area of each mouse in each group, the lengths of the hairs were measured using a vernier caliper, and the lengths of the hairs of the mice in each group were counted. The skin of the depilated area of the mice in each group was cut out, and hematoxylin-eosin staining (HE staining) was performed. The morphology of the hair follicles was observed under a microscope. 6 fields (×100) were randomly selected for each sample to be observed, and the average number of hair follicles in each field was calculated. The software used for data statistics was Graph Pad 8.0, and the data of the groups were expressed as mean ± standard deviation (X ± SD), as shown in Table 5 below. Photographs of hair growth and representative HE-stained sections of the skin tissues from representative mice in each group on day 18 are shown in FIG. 3.

**Table 5. Hair length and number of hair follicles for different groups (expressed as X ± SD)**

| Group | Daily dose of administration | Hair length (X ± SD, mm) | Number of hair follicles (X ± SD) |
|---|---|---|---|
| Blank group | 0 | 6.035±0.038 | 110.00±41.867 |
| Model group | 0 | 0.948±0.090 | 18.20±2.615 |
| Positive control drug group | 37.5 mg of clascoterone | 5.649±0.059 | 53.533±24.929 |
| Compound 7 group | 1.5 mg of compound 7 | 3.850±0.009 | 95.600±26.091 |
| Compound 8 group | 1.5 mg of compound 8 | 4.493±0.024 | 116.733±35.809 |

FIG. 3 shows that compared to the blank group, the mice in the model group had poor hair growth and significantly fewer hair follicles than the blank group, indicating that coating 0.5% testosterone propionate solution may result in an alopecia mouse model; compared to the model group, the mice in the positive control drug group had significantly grown hairs and the number of hair follicles was significantly increased, indicating that the alopecia mouse model is reliable; under the condition that the daily dose of administration of compound 7 and compound 8 was 1.5 mg, the growth of hairs and hair follicles was better than that of the positive control drug group, indicating that the compounds of the present disclosure at a low dose have better alopecia treatment effects than the positive control drug at a high dose.

As can be seen from the above test data, the compounds of the present disclosure can significantly increase the hair length and the number of hair follicles of mice in the test groups at a relatively small dose of administration, can promote the generation of the hair follicles and the growth of the hair in the mice with alopecia, and have good hair growth-promoting effects.

The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

## Claims

1. A compound represented by formula I, a racemate thereof, a stereoisomer thereof, a tautomer thereof, a solvate thereof, a polymorph thereof or a pharmaceutically acceptable salt thereof, 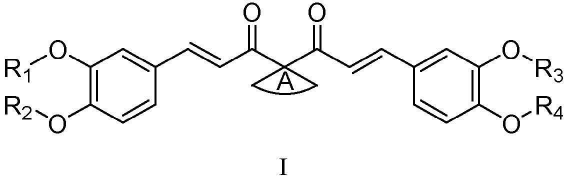
wherein R₁, R₂, R₃, and R₄ are identical or different and are each independently selected from the following groups that are unsubstituted or optionally substituted with one, two or more Ra: C₁₋₁₂ alkyl or deuterated C₁₋₁₂ alkyl;
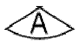 represents the following ring systems that are unsubstituted or optionally substituted with one, two or more Rb: C₃₋₂₀ cycloalkyl, C₄₋₂₀ cycloalkenyl, 3- to 20-membered heterocyclyl, a spiro ring formed from C₃₋₂₀ cycloalkyl and C₃₋₂₀ cycloalkyl, a spiro ring formed from C₃₋₂₀ cycloalkyl and 3- to 20-membered heterocyclyl, and a spiro ring formed from 3- to 20-membered heterocyclyl and 3- to 20-membered heterocyclyl;
Ra are identical or different and are each independently selected from halogen, =O, hydroxyl, amino, C₁₋₁₂ alkyl, and C₁₋₁₂ alkoxy;
Rb are identical or different and are each independently selected from deuterium, halogen, =O, hydroxyl, amino, and the following groups that are unsubstituted or optionally substituted with one, two or more Rc: C₁₋₁₂ alkyl, C₁₋₁₂ alkoxy, C₃₋₂₀ cycloalkyl, 3- to 20-membered heterocyclyl, -NHC₁₋₁₂ alkyl, -N(C₁₋₁₂ alkyl)₂, -NHCOC₁₋₁₂ alkyl, -CONHC₁₋₁₂ alkyl, -NHC₃₋₂₀ cycloalkyl, -NHCOC₃₋₂₀ cycloalkyl, -CONHC₃₋₂₀ cycloalkyl, -S(O)₂C₁₋₁₂ alkyl, and -COOC₁₋₁₂ alkyl; or two Rb attached to the same carbon atom, together with the carbon atom attached thereto, form the following ring systems that are unsubstituted or optionally substituted with one, two or more Rc: C₃₋₂₀ cycloalkyl and 3- to 20-membered heterocyclyl;
Rc is selected from halogen, hydroxyl, amino, C₁₋₁₂ alkyl, C₁₋₁₂ alkoxy, C₃₋₂₀ cycloalkyl, 3- to 20-membered heterocyclyl, and 5- to 20-membered heteroaryl.

2. The compound according to claim 1, wherein R₁, R₂, R₃, and R₄ are identical or different and are each independently selected from C₁₋₆ alkyl and deuterated C₁₋₆ alkyl;
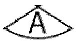 represents the following ring systems that are unsubstituted or optionally substituted with one, two or more Rb: C₃₋₁₂ cycloalkyl, C₄₋₁₂ cycloalkenyl, 3- to 12-membered heterocyclyl, a spiro ring formed from C₃₋₁₂ cycloalkyl and C₃₋₁₂ cycloalkyl, a spiro ring formed from C₃₋₁₂ cycloalkyl and 3- to 12-membered heterocyclyl, and a spiro ring formed from 3- to 12-membered heterocyclyl and 3- to 12-membered heterocyclyl;
Rb are identical or different and are each independently selected from deuterium, halogen, =O, hydroxyl, amino, and the following groups that are unsubstituted or optionally substituted with one, two or more Rc: C₃₋₁₂ cycloalkyl, 3- to 12-membered heterocyclyl, -NHC₁₋₆ alkyl, -N(C₁₋₆ alkyl)₂, - NHCOC₁₋₆ alkyl, -NHC₃₋₁₂ cycloalkyl, -S(O)₂C₁₋₆ alkyl, and -COOC₁₋₆ alkyl;
Rc is selected from halogen, hydroxyl, C₃₋₁₂ cycloalkyl, 3- to 12-membered heterocyclyl, and 5- to 12-membered heteroaryl.

3. The compound according to claim 1 or 2, wherein 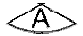 is selected from the following ring systems that are unsubstituted or optionally substituted with one, two or more Rb: cyclopropyl, cyclobutyl, cyclopentyl, cyclohexyl, cyclohexenyl, spiro[2.3]hexanyl, 1,3-dioxanyl, and 1,4-dioxaspiro[4,5]decanyl;
Rb is selected from deuterium, F, Cl, Br, I, =O, hydroxyl, amino, tert-butoxycarbonyl, -S(O)₂CH₃, - COOC(CH₃)₃, 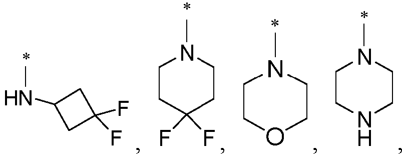 -N(C₂H₅)₂, -N(CH₃)₂, 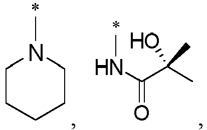 and 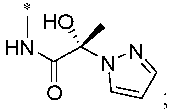
R₁, R₂, R₃, and R₄ are identical or different and are each independently selected from C₁₋₃ alkyl and deuterated C₁₋₃ alkyl.

4. The compound according to any one of claims 1-3, wherein the compound represented by formula I is selected from the following:

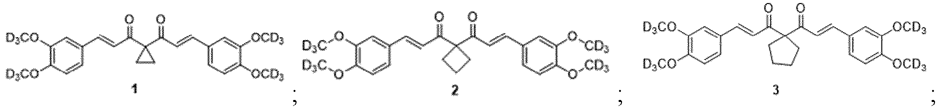

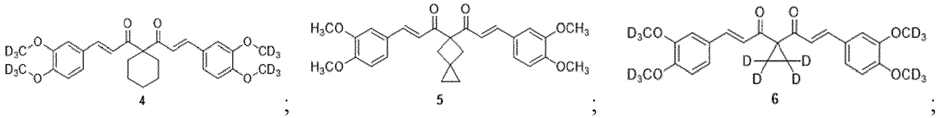

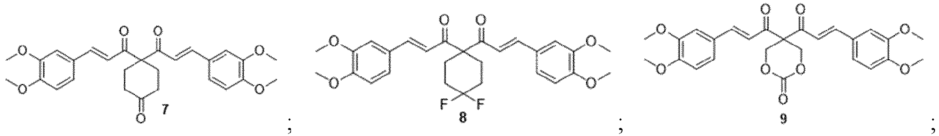

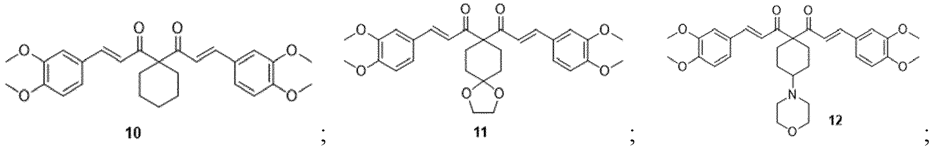

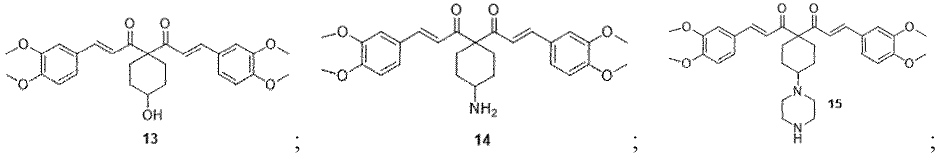

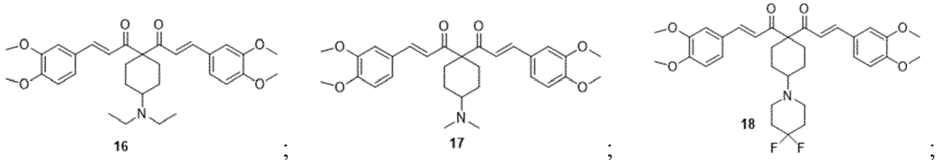

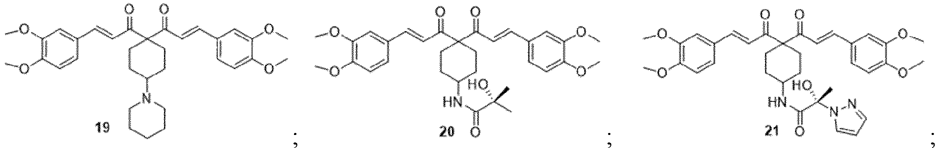

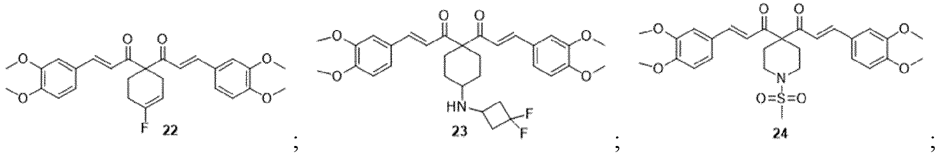

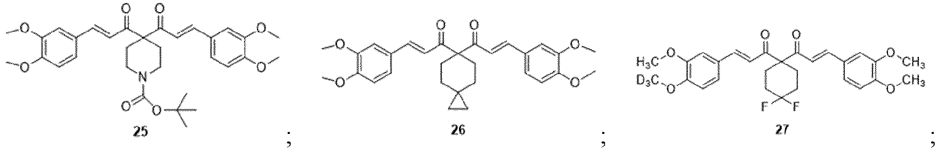

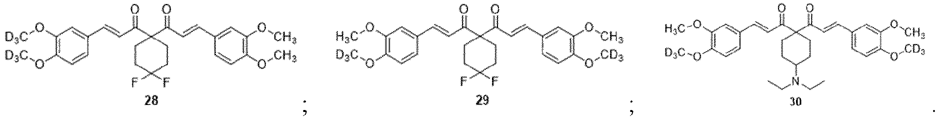

5. The compound according to any one of claims 1-4, wherein the compound represented by formula I is selected from the following:

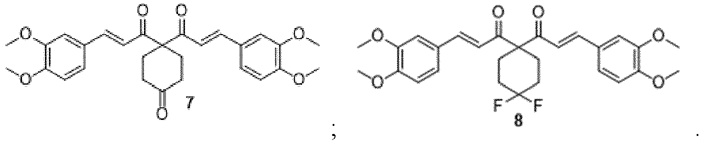

6. The compound according to any one of claims 1-5, wherein the pharmaceutically acceptable salt of the compound represented by formula I is hydrochloride thereof.

7. A pharmaceutical composition, comprising at least one of the compound represented by formula I, the racemate thereof, the stereoisomer thereof, the tautomer thereof, the solvate thereof, the polymorph thereof or the pharmaceutically acceptable salt thereof according to any one of claims 1-6.

8. The pharmaceutical composition according to claim 7, further comprising one or more pharmaceutically acceptable auxiliary materials;
preferably, suitable routes of administration for the pharmaceutical composition include: oral, rectal, topical, buccal, parenteral, intramuscular, intradermal, intravenous, and transdermal administration; preferably, the pharmaceutical composition is for topical administration, and the pharmaceutical composition can be an ointment, a cream, a paste, a tincture, a plaster, a gel, a film, a paint, an aerosol, a spray, a foam, or a microsponge.

9. Use of the compound represented by formula I, the racemate thereof, the stereoisomer thereof, the tautomer thereof, the solvate thereof, the polymorph thereof or the pharmaceutically acceptable salt thereof according to any one of claims 1-6, or the pharmaceutical composition according to claim 7 or 8 for manufacturing a medicament for the treatment, prevention, or amelioration of symptoms or diseases of androgen-related disorders.

10. The use according to claim 9, wherein the symptoms or diseases of androgen-related disorders are selected from: androgen-related inflammation, including wounds, acne, atopic dermatitis, rheumatoid arthritis, psoriasis, and rosacea; polyglutamine-mediated degeneration of motor neurons, and Kennedy's disease (spinal and bulbar muscular atrophy or SBMA); androgen-related cancers such as prostate cancer, bladder cancer, breast cancer, ovarian cancer, endometrial cancer, hepatocellular carcinoma, hepatocellular carcinoma, central nervous system cancer, skin cancer, lymphoma, leukemia, esophageal cancer, gastric cancer, colon cancer and pancreatic cancer; alopecia, including androgenetic alopecia; comedone; and hirsutism.
